# EUROPÄISCHE PATENTSCHRIFT

(11) **EP 2 906 577 B1**
(45) Veröffentlichungstag und Bekanntmachung des Hinweises auf die Patenterteilung: **22.01.2020**
(21) Anmeldenummer: 13779194.3
(22) Anmeldetag: 15.10.2013
(51) Int. Cl.: C07H 15/252, C07H 15/08, C07D 317/32, C07D 317/34, C07C 15/28, C07D 307/20, A61K 31/7034, A61P 35/00

(54) **ANTHRACYCLIN-DERIVATE ZUR BEHANDLUNG VON TUMORERKRANKUNGEN**
ANTHRACYLINE DERIVATIVES FOR TREATING TUMOR DISEASES
DÉRIVÉS D'ANTHRACYCLINE POUR LE TRAITEMENT DE PATHOLOGIES TUMORALES

(30) Priorität: 15.10.2012 AT 11142012
(43) Veröffentlichungstag der Anmeldung: 19.08.2015
(73) Patentinhaber: Produkem Molekulares Design GmbH, 1180 Wien (AT)
(72) Erfinder: NOE, Christian R., A-1180 Wien (AT); SONNTAGBAUER, Michael, A-1140 Wien (AT); QUEVA, Sébastien, F-95310 Saint Ouen L Aumone (FR); URBAN, Ernst, A-2380 Perchtoldsdorf (AT)
(74) Vertreter: Sommer, Andrea
(86) Internationale Anmeldenummer: PCT/EP2013/071520
(87) Internationale Veröffentlichungsnummer: WO 2014/060408

(56) Entgegenhaltungen:
- EP-A1- 0 523 289
- CA-A2- 1 131 622
- DE-A1- 19 708 496
- US-B1- 6 355 784
- F. ARCAMONE ET AL: "Stereocontrolled glycosidation of daunomycinone. Synthesis and biological evaluation of 6-hydroxy-L-arabino analogues of antitumor anthracyclines", JOURNAL OF MEDICINAL CHEMISTRY, Bd. 19, Nr. 5, 1. Mai 1976 (1976-05-01), Seiten 733-734, XP055090144, ISSN: 0022-2623, DOI: 10.1021/jm00227a033 in der Anmeldung erwähnt
- KEN NAKAI ET AL: "Synthesis and antitumor activity of 5'-demethyl-5'-trifluoromethyl-daunorubici n and -doxorubicin", CARBOHYDRATE RESEARCH, Bd. 320, Nr. 1-2, 1. Juli 1999 (1999-07-01), Seiten 8-18, XP055090146, ISSN: 0008-6215, DOI: 10.1016/S0008-6215(99)00126-3 in der Anmeldung erwähnt
- A.B. ARGADE ET AL: "Marschalk reaction approach for a simple synthesis of ( )4-demethoxydaunomycinone", TETRAHEDRON LETTERS, Bd. 27, Nr. 30, 1. Januar 1986 (1986-01-01), Seiten 3529-3532, XP055097184, ISSN: 0040-4039, DOI: 10.1016/S0040-4039(00)84841-8

## Beschreibung

Anthracycline kommen in großem Umfang bei einem breiten Spektrum von Tumorerkrankungen zum Einsatz. Ihre klinische Anwendung ist jedoch durch dosisabhängige Nebenwirkungen und auftretende Tumorresistenzen stark limitiert. Um diese Problematik zu umgehen werden bisher zwei grundsätzliche Strategien verfolgt.

Einerseits wird durch Techniken des "Drug Targeting" versucht, die Anreicherung bzw. Freisetzung der Wirkstoffe im Tumorgewebe zu erhöhen. Zum Einsatz kommen dabei Prodrugs, wobei die Anthracycline mit Peptiden, Kohlenhydraten, Antikörpern oder synthetischen Polymeren verknüpft werden, und spezielle vor allem liposomale Formulierungen der Arzneistoffe. Beispiele dafür sind in der Literatur ausführlich beschrieben (Krohn K. "Anthracycline Chemistry and Biology II" Topics in current chemistry, 283 (2008), 73-140).

Die zweite Strategie ist das Design neuer Anthracyclinwirkstoffe. Da herkömmliche Anthracycline im Regelfall rein fermentativ gewonnen werden, bleiben die Möglichkeiten chemischer Modifikation begrenzt und beschränken sich auf wenige funktionelle Gruppen. Modifikationen sind außerdem nur an bestimmten Positionen sinnvoll, da die planare Struktur des Moleküls erhalten bleiben muss, um die DNA interkalierende Wirkung zu erzielen. Die am häufigsten für Modifikationen genutzten funktionellen Gruppen sind am Aglycon die Hydroxygruppe an Position 14, die Ketogruppe an Position 13 und am Zuckerteil die 3'- Aminogruppe bzw. die 4'-Hydroxygruppe.

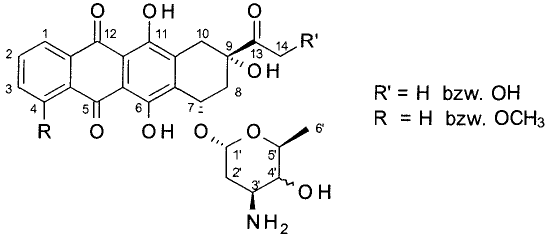

### Anthracyclin-Grundstruktur

Beispiele für derartige Modifikationen sind Anthracyclin-Disacharide darunter Sabarubicin welches bereits klinisch getestet wurde (M. Bigioni et al., Antitumour effect of combination treatment with Sabarubicin (MEN 10755) and cisplatin (DDP) in human lung tumour xenograft, Cancer chemotherapy and pharmacology, 62 (2008) 621-629.). Weitere bekannte Beispiele für zuckermodifizierte Anthracycline sind Derivate die eine Morpholino-Struktur enthalten. Der bekannteste Vertreter ist Nemorubicin, welches ebenfalls klinisch getestet wurde (C. Sessa et al., Ongoing phase I and II studies of novel anthracyclines, Cardiovascular toxicology, 7 (2007) 75-79.).

EP 0 523 289 A1 offenbart ein Verfahren zur Synthese von Doxorubicin und dessen Derivate, durch eine neue Synthese von Daunomycinon und seiner Derivate (siehe Seite 2, Zeilen 1 bis 3). Sowohl Doxorubicin als auch Daunomycinon weisen eine unsubstituierte Methylgruppe in Position 6 des Zuckerrestes auf.

DE 197 08 496 A1 betrifft die Synthese von Aglyconen, die in Position 7 durch eine OH-Gruppe oder eine Alkoxygruppe substituiert sind (siehe Anspruch 1).

CA 1,131,622 A offenbart 4-demethoxydaunorubicin, welches als Zucker eine L-Daunosamin-Gruppe in Position 7 aufweist (siehe Seite 6) und seine Synthese. Die Position 6 der L-Daunosamin-Gruppe weist lediglich eine Methylgruppe auf. A. B. Argade et all offenbaren in Tetrahedron Letters Vol. 27, Nr.30 Seiten 3529 to 3532 (1986) eine neues Synthese eines Aglycons. Auch hier ist als Zuckerrest L-Daunosamin vorgesehen.

Gegenstand der vorliegenden Erfindung sind neue Anthracycline, deren physikochemische Eigenschaften, vor allem Basizität und hydrophiles-lipophiles Gleichgewicht durch gezielte Strukturmodifikationen so verändert werden, dass sich ihre pharmakodynamischen bzw. pharmakokinetischen Eigenschaften gezielt ändern. Da strukturelle Veränderungen eines Wirkstoffes die Wirkstoff-Rezeptor-Interaktion massiv stören können, ist die Auswahl jener Stelle, an welcher die strukturelle Veränderung erfolgt, von besonderer Bedeutung. Bei den Verbindungen der vorliegenden Erfindung werden die strukturellen Veränderungen unter anderem an der Position 6 des Zuckerteils vorgenommen. Diese Position ist in den therapeutisch angewandten Anthracyclinen durch eine Methylgruppe besetzt. Durch Röntgenkristallographische Aufnahmen wurde gezeigt, dass bei den als Interkalierern wirkenden Anthracyclinen, dieser Zuckerteil in der "minor groove" der DNA positioniert ist (C.A. Frederick et al., Structural comparison of anticancer drug-DNA complexes: adriamycin and daunomycin, Biochemistry, 29 (1990) 2538-2549.). Durch die Positionierung des Struktur-modifizierenden Teiles an die Position 6 des Zuckerringes, kann eine Störung der Struktur-Wirkungsinteraktion vermieden werden. Selbst sterisch anspruchsvolle Reste können sich in den Raum um das Molekül herum erstrecken, ohne die Interkalierung zu behindern. Durch die räumliche Nähe zu den sauren Phosphatgruppen der DNA in der "minor groove" können darüber hinaus basische Reste leicht mit diesen interagieren.

Ein Charakteristikum der Strukturmodifikationen gemäß der vorliegenden Erfindung besteht darin, dass die Reste, welche die Eigenschaften des Wirkstoffes modulieren, über ein Sauerstoff-Stickstoff- oder Schwefelatom an die Position 6 des Zuckers gebunden sind. Bisher sind in der Literatur lediglich halogenierte Derivate an dieser Position beschrieben, sowie Verbindungen mit einer freien Hydroxy- bzw. Aminogruppe.

F. Arcamone, et al., Stereocontrolled glycosidation of daunomycinone. Synthesis and biological evaluation of 6-hydroxyL-arabino analogues of antitumor anthracyclines, Journal of Medicinal Chemistry, 19 (1976) 733-734.

K. Nakai, et al., Synthesis and antitumor activity of 5'-demethyl-5'-trifluoromethyl-daunorubicin and -doxorubicin, Carbohydr. Res., 320 (1999) 8-18.

E.F. Fuchs, et al., New daunorubicin analogs. 3-Amino-2,3,6-trideoxy-α- and β-D-arabino- and 3,6-diamino-2,3,6-trideoxy-α-D-ribo-hexopyranosides of daunomycinone, Journal of Antibiotics, 32 (1979) 223-238.

Patent US 6355784 " Methods and compositions for the manufacture of halogenated anthracyclines with increased antitumor activity, other anthracyclines, halogenated sugars, and glycosyl donors", filed June 1999, issued March 2002.

Da eine Methylgruppe, wie bei den Zuckerteilen der therapeutisch eingesetzten Anthracycline, nämlich Acosamin und Daunosamin, nicht mit bekannten synthetischen Methoden funktionalisiert werden kann, sind auch die neuen bisher unbekannten Zucker und ihre Derivate, sowie deren Herstellung, die derartige 6'-Modifikation zugänglich macht und deren Verwendung Gegenstand der Erfindung.

Gegenstand der vorliegenden Erfindung sind neue Anthracyclinderivate der allgemeinen Formel (I), 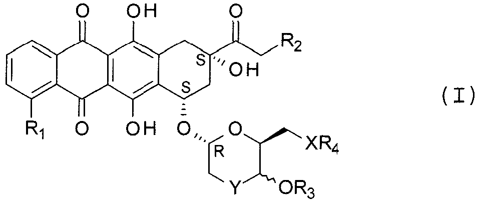 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₂ ein Wasserstoffatom, eine Hydroxy- oder Methoxygruppe, ein Acyl- oder Aroylrest, vorzugsweise Acetyl oder Benzoyl ist; R₃ Wasserstoff, Trifluoracetyl (C(=O)CF₃) oder *p*-Nitrobenzoyl (C(=O)PhNO₂) ist und die gewellte Linie jeweils beide möglichen Konfigurationen von -OR₃ in Bezug zum Grundkörper bedeutet; Y= [C(=O)], C(=N)-OH] oder [CH-OH], [CH-NR₅R₆] in beiden möglichen stereoisomeren Anordnungen bedeutet, vorzugsweise jedoch [CH-NR₅R₆] bedeutet, wobei R₅ und R₆ bevorzugt gleich sind und für jeweils ein Wasserstoffatom stehen, jedoch auch verschieden sein können und für ein Wasserstoffatom oder eine Aminoschutzgruppe, vorzugsweise einer Trifluoracetylgruppe (TFA) stehen; worin X = O, S oder NR, mit R= Wasserstoff oder C₁ bis C₄ Alkyl, vorzugsweise Methyl, Ethyl, Propyl und tert. Butyl oder n-Butyl, bedeutet, am meisten bevorzugt Methyl oder Ethyl; worin R₄ eine unverzweigte oder verzweigte Alkyl- oder Heteroalkylkette mit einer Kettenlänge von 1 bis 19 Elementen bedeutet, wobei die maximal 6 Heteroatome (O, N, S) beliebig kombinierbar durch mindestens zwei Kohlenstoffatome voneinander getrennt sind. Vorzugsweise ist R₄ eine Gruppe (CH₂-CH₂-O)ₙ- mit n = 1 bis 6, wobei am terminalen Sauerstoffatom der (CH₂-CH₂-O)ₙ-Gruppe ein Wasserstoffatom oder eine C₁ bis C₄ Alkylgruppe, vorzugsweise Methyl, Ethyl, Propyl und tert. Butyl oder n-Butyl angebracht ist, am meisten bevorzugt eine Methyl- oder Ethylgruppe, gebunden ist.

Geeignete Schutzgruppen, insbesondere für die Aminogruppe sind dem Fachmann aus dem Stand der Technik bekannt beispielsweise aus "Protective Groups in Organic Synthesis" (Greene, Wuts) 4. Auflage, John Wiley&Sons, Inc., Seiten 781 bis 783.

In einer bevorzugten Ausführungsform, gemäß Formel (Ia), 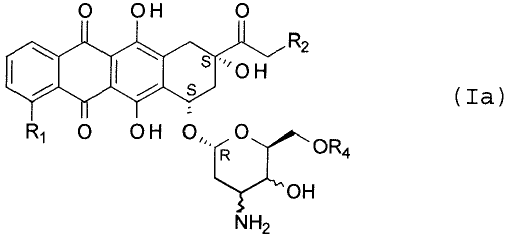 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₂ ein Wasserstoffatom, eine Hydroxy- oder Methoxygruppe, ein Acyl- oder Aroylrest, vorzugsweise Acetyl oder Benzoyl ist; die Aminogruppe als auch die Hydroxygruppe in beiden möglichen stereochemischen Anordungen vorliegen kann, R₄ die Bedeutung -(CH₂-CH₂-O)ₙ- mit n= 1 bis 6, vorzugsweise n= 3, 4 oder 5 bedeutet und worin am terminalen Sauerstoffatom der Kette ein Wasserstoffatom oder eine C₁ bis C₄ Alkylgruppe, vorzugsweise Methyl, Ethyl, Propyl und tert. Butyl oder n-Butyl angebracht ist, am meisten bevorzugt eine Methylgruppe oder Ethylgruppe gebunden ist.

Ein weiterer Gegenstand der vorliegenden Erfindung ist ein Verfahren zur Herstellung der Anthracyclinderivate der allgemeinen Formel(I), wobei eine offenkettige Zuckerverbindung der allgemeinen Formel (II), 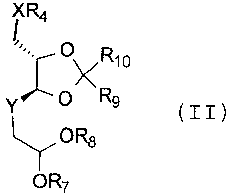 in welcher R₇ und R₈ gleich sind und für Alkyl oder Alkylen mit 2 bis 3 C-Atomen stehen; R₉ und R₁₀ für eine Alkylgruppe mit 1 bis 3 C-Atomen stehen; X, Y und R₄ die in Formel (I) gegebene Bedeutung haben, zyklisiert wird. Damit eine Verbindungen der allgemeinen Formel (II) intramolekular zyklisiert, muss die Diol- und Aldehyd-Schutzgruppe gespalten werden, dies geschieht unter sauren Bedingungen, vorzugsweise in einer Mischung von Trifluoressigsäure in Tetrahydrofuran/Wasser als Lösungsmittel bei einer Temperatur von ungefähr 20°C bis 100°C, vorzugsweise bei 55 bis 65°C. Die daraus resultierende Pyranose der allgemeinen Formel (III), 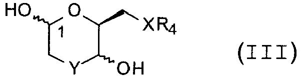 in welcher X, Y und R₄ die in Formel (I) gegebene Bedeutung haben, entsteht als Gemisch der beiden Anomere an Position 1 der allgemeinen Formel (III), welches nicht weiter aufgetrennt werden muss, da sich beide Anomere für die Glycosylierung eignen.

In weiterer Folge werden Verbindungen der allgemeinen Formel (III) mit einem tetracyklischen Aglycon vom Strukturtyp der Anthracycline (AA) glykosyliert. Bevorzugt ist AA ein interkalierungsfähiges tetracyklisches Aglykon vom Strukturtyp der Anthracycline.

Als Glykosylierungsreaktion können eine Reihe von Reaktionen verwendet werden. Die Verbindung III kann direkt in der Reaktion eingesetzt werden oder kann vorzugsweise vorher für die Glykosylierung aktiviert werden. Diese Aktivierung wird vorzugsweise mit p-Nitrobenzoylchlorid in Pyridin bei einer Temperatur von -10°C bis 50°C, vorzugsweise bei ungefähr 0°C durchgeführt. Diese Reaktion führt zu Verbindungen der allgemeinen Formel (IIIa) führt. Gut geeignet zur Aktivierung ist ebenso Trifluoressigsäureanhydrid in Diethylether, wobei die Reaktion bei von -10°C bis 50°C vorzugsweise bei ungefähr 0°C durchgeführt wird und zu Verbindungen der allgemeinen Formel (IIIb),

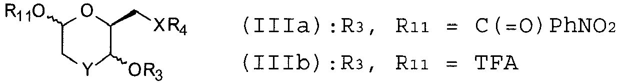

In IIIa und IIIb haben X, Y und R₄ die in Formel (I) gegebene Bedeutung.

Die Glycosylierung mit einem tetracyklischen Aglycon vom Strukturtyp der Anthracycline (AA) führt zu einer Verbindung der allgemeinen Formel IIIc, 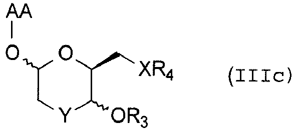 in welcher AA ein tetracyclisches Aglycon vom Strukturtyp der Anthracycline ist, vorzugsweise ein interkalierungsfähiges tetrazyklisches Aglycon und X, Y, R₃ und R₄ die in Formel I angegebene Bedeutung haben.

Die anschließende Abspaltung der Schutzgruppen, kann nach in der Literatur ausführlich beschriebenen Methoden durchgeführt werden (T. Matsumoto, M. Osaki, K. Yamada, F. Matsuda, S. Terashima, 14-Fluoroanthracyclines. Novel syntheses and antitumor activity, Chemical & Pharmaceutical Bulletin, 36 (1988) 3793-3804);(Y. Kimura, M. Suzuki, T. Matsumoto, R. Abe, S. Terashima, Novel glycosidation of 4-demethoxyanthracyclinones by the use of trimethylsilyl triflate. Syntheses of optically active 4-demethoxydaunorubicin and 4-demethoxyadriamycin, Bulletin of the Chemical Society of Japan, 59 (1986) 423-431).

In der bevorzugten Ausführungsform werden die aktivierten Pyranosen der allgemeinen Formel (IIIa) oder (IIIb) mit einem vom Anthrachinon abgeleiteten Aglycon der allgemeinen Formel (IV), 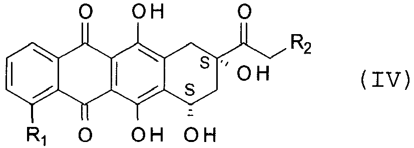 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₂ ein Wasserstoffatom, eine Hydroxy- oder Methoxygruppe, ein Acyl- oder Aroylrest, vorzugsweise Acetyl oder Benzoyl ist, zur Glykosidierungsreaktion gebracht, wobei eine Reaktionsaktivierung erfolgt. Beispielsweise wird zur Reaktionsaktivierung Trimethylsilyltrifluoromethanesulfonate (TMSOTf) in einer Mischung aus Dichlormethan/Diethylether bei Temperaturen von ungefähr 0 bis -70°C, vorzugsweise zwischen 0 und -20°C verwendet. Die am Zucker noch vorhandenen Schutzgruppen [TFA, C(=O)PhNO₂,] können unter basischen Bedingungen, vorzugsweise mit Natronlauge abgespalten werden, wobei Verbindungen der allgemeinen Formel (Ia) erhalten werden. 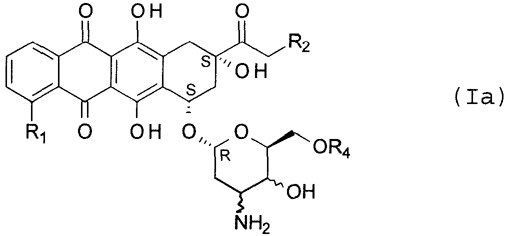 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₂ ein Wasserstoffatom, eine Hydroxy- oder Methoxygruppe, ein Acyl- oder Aroylrest, vorzugsweise Acetyl oder Benzoyl ist, R₄ die Bedeutung -(CH₂-CH₂-O)ₙ- mit n= 1 bis 6, vorzugsweise n= 3, 4 und 5 hat und worin am terminalen Sauerstoffatom der Kette ein Wasserstoffatom oder eine C₁ bis C₄ Alkylgruppe, vorzugsweise Methyl, Ethyl, Propyl und tert. Butyl oder n-Butyl, bedeutet, am meisten bevorzugt eine Methylgruppe oder Ethylgruppe angebracht ist.

Die offenkettigen Zuckerverbindungen der allgemeinen Formel (II), stellen gemeinsam mit den Verfahren zu ihrer Herstellung einen weiteren Gegenstand der vorliegenden Erfindung dar.

### Offenkettige Zuckerverbindungen der allgemeinen Formel (II)

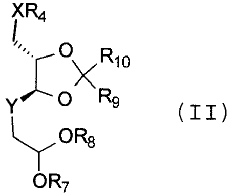

können durch Totalsynthese hergestellt werden, wobei vorzugsweise die Addition eines C2-Bausteins der allgemeinen Formel (V), 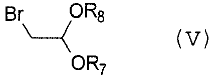 in welcher R₇ und R₈ die in Formel (II) gegebene Bedeutung haben, mit einem geschützten Derivat der L-Threose (enantiomerenreiner C4-Baustein) der allgemeinen Formel (VI), 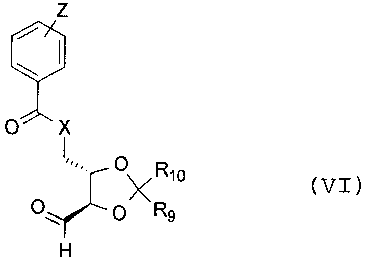 in welcher X, R₉, R₁₀ die in Formel (II) gegebene Bedeutung haben und Z = Wasserstoff, oder mindestens oder mehrere, vorzugsweise eine oder zwei, Methyl-, Fluor-, Chlor-, Brom- oder Nitrogruppen bedeutet, angewandt wird.

Verbindungen der allgemeinen Formel (VI) in welcher X= O und R₉ bzw. R₁₀ eine Methylgruppe ist, sind in der Literatur ausführlich beschrieben (S. Morgenlie, Identification of the products of periodate oxidation of some mono-O-isopropylidene derivatives of aldoses and alditols by g.l.c.-m.s, Carbohydr. Res., 138 (1985) 329-334); (N. Cohen, B.L. Banner, R.J. Lopresti, Synthesis of optically active leukotriene (SRS-A) intermediates, Tetrahedron Letters, 21 (1980) 4163-4166).

Die C-C Knüpfung zwischen Verbindungen der allgemeinen Formel (V) und Verbindungen der allgemeinen Formel (VI) erfolgt bevorzugt durch eine Grignard-Reaktion, vorzugsweise in einem aprotischen Lösungsmittel wie Tetrahydrofuran, Methyl tert-butyl ether oder Diethylether bei einer Temperatur von ungefähr 0°C bis 100°C, vorzugsweise bei Raumtemperatur, wobei Verbindungen der allgemeinen Formel (VII), 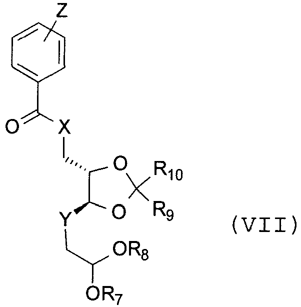 in welcher R₇, R₈, R₉, R₁₀ die in Formel (II) gegebene Bedeutung haben; X, R₉, R₁₀ die in Formel (II) gegebene Bedeutung haben und Z = Wasserstoff, oder mindestens oder mehrere, vorzugsweise eine oder zwei, Methyl-, Fluor-, Chlor-, Brom- oder Nitrogruppen bedeutet und Y= CH-OH in beiden möglichen stereoisomeren Anordnungen bedeutet, entstehen.

Dieses Additionsprodukt wird anschließend zum Keton [Y= C(=O)] der allgemeinen Formel (VII) oxidiert, vorzugsweise durch eine Swern-Oxidation in Dichlormethan bei tiefen Temperaturen, vorzugsweise bei -50°C bis -90°C, am meisten bevorzugt bei -60°C durchgeführt.

Zur Einführung des Stickstoffs in das Molekül stehen verschiedene Methoden zur Verfügung. Am besten kann diese Reaktion durch Umsetzung des Ketons [Y= C(=O)] der allgemeinen Formel (VII) zu einem Oxim [Y= C(=N)-OH] der allgemeinen Formel (VII) erfolgen, vorzugsweise wird diese Reaktion mit Hydroxylaminhydrochlorid durchgeführt, vorzugsweise in Pyridin bei 20°C bis 80°C, am meisten bevorzugt bei ungefähr 55°C.

In weiterer Folge wird die Aroylschutzgruppe an X abgespalten, vorzugsweise in basischem Medium, vorzugsweise mittels NaOH in THF/H₂O. Nach der Abspaltung wird ein Rest R4 gemäß der in Formel I gegebenen Bedeutung eingeführt, wobei die Einführung einer unverzweigten oder verzweigten Alkyl- oder Heteroalkylkette an X vorzugsweise durch nukleophile Substitution, besonders bevorzugt mithilfe einer nicht nukleophilen Base, wie Natriumhydrid in THF oder DMF erfolgt und der einzuführende Rest R4 zuvor aktiviert wird. Zur Aktivierung wird bevorzugt ein Tosylatrest als Abgangsgruppe verwendet, beispielsweise para-Toluolsulfonsäurechlorid, NaOH und Tetrahydrofuran/Wasser bei Temperaturen von 0°C bis 60°.

Das daraus resultierende Oxim [Y= C(=N)-OH] der allgemeinen Formel (II) wird zum Amin [Y= CH-NH₂] der allgemeinen Formel (II)reduziert. Für diese Reaktion stehen eine Vielzahl von Reagentien zur Verfügung, wobei komplexe chirale und nicht chirale Matallhydride, wie Natrium-bis-(2-methoxy-ethoxy)-aluminium-dihydrid oder LiAlH₄ besonders gut geeignet sind. Vorzugsweise wird diese Reaktion mit Natrium-bis-(2-methoxy-ethoxy)-aluminium-dihydrid oder LiAlH₄ in Toluol oder THF bei 0°C bis 100°C, vorzugsweise bei Raumtemperatur durchgeführt.

Die erhaltene Aminogruppe wird ihrerseits mit einer Schutzgruppe versehen, welche zur selektiven Abspaltung nach Ringschluß und Glykosylierung geeignet ist, wobei für diesen Zweck die Trifluoracetyl-Gruppe besonders gut geeignet ist und die Reaktion mit Trifluoressigsäureanhydrid in Pyridin als Lösungsmittel durchgeführt werden kann, vorzugsweise bei 0°C bis 60°C, vorzugsweise bei Raumtemperatur und so eine Zuckerverbindung der allgemeinen Formel (II) [Y= CH-NR₅R₆] entsteht, in welcher R₅/R₆ verschieden sind und die in Formel (I) angegebene Bedeutung haben.

Kein Gegenstand der vorliegenden Erfindung ist ein Verfahren zur Herstellung von vom Anthrachinon abgeleiteter Aglycone der allgemeinen Formel (IV) 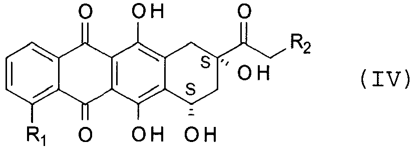 welche durch Totalsynthese hergestellt werden, wobei in ihrer bevorzugten Ausführungsform die stereoselektive Alkylierung basierend auf der Seebach-Reaktion (Seebach, D.; Sting, A. R.; Hoffmann, M. Angewandte Chemie, International Edition in English 1997, 35, 2708) durchgeführt wird, während bei der Zyklisierung die Marschalk-Reaktion (Marschalk, C.; Koenig, F.; Ouroussoff, N. Bulletin de la Societe Chimique de France, Memoires 1936, 3, 1545) zur Anwendung kommt.

Das Ringsystem wird durch die Umsetzung eines trizyklischen Bromids der allgemeinen Formel (VIII), 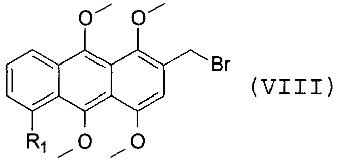 in welcher R₁ die in Formel (I) angegebene Bedeutung hat, mit einem Synthon der allgemeinen Formel (IX), 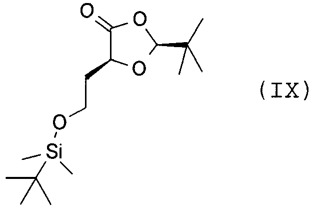 hergestellt, wobei die Verbindung der allgemeinen Formel (IX) zunächst mit einer nicht-nukleophilen Base, wie beispielsweise LDA (Lithiumdiisopropylamid), LiHMDS (Lithiumbis(trimethylsilyl)-amid) vorzugsweise jedoch mit KHMDS (Kaliumbis(trimethylsilyl)-amid) in einem polaren aprotischen Lösungsmittel (z.B. Dimethylformamid oder Tetrahydrofuran) deprotoniert wird. Die Reaktion erfolgt vorzugsweise bei -40°c bis -90°C, am meisten bevorzugt bei -60°C bis -80°C, am allermeisten bevorzugt bei -76°C.

Die darauffolgende Alkylierung führt zur Bildung einer enantiomerenreinen Verbindung der allgemeinen Formel (X), 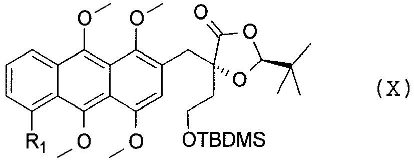 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist.

Die Umsetzung einer Verbindung der allgemeinen Formel (X) mit einem organometallischen Reagens wie Organo-Magnesium, -Lithium oder -Natrium bevorzugt mit Methyl Lithium in einen aprotischen Lösungsmittel wie z.B. Tetrahydrofuran bei -40°C bis -90°C, bevorzugt bei -50°C bis -80°C, am meisten bevorzugt bei -78°C, ergibt eine Verbindung der Formel (XI), 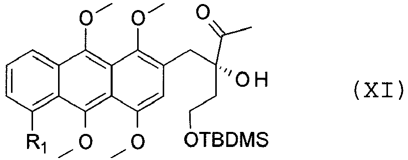 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist.

Die Ketogruppe der Verbindung der allgemeinen Formel (XI) wird in einer Reduktionsreaktion, vorzugsweise einer Hydridreduktion z.B. mit Natriumborhydrid in Ethanol als Lösungsmittel vorzugsweise bei 10°C bis 70°C, vorzugsweise bei Raumtemperatur, zu einer Hydroxylgruppe reduziert und anschließend zu einer Verbindung der allgemeinen Formel (XII), 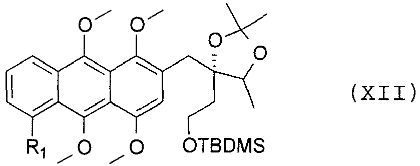 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist, ketalisiert.

Die Ketalisierung kann vorzugsweise mit Dimethoxypropan und p-Toluolsulfonsäure in Aceton durchgeführt werden. Nach der Spaltung der Silyl-Schutzgruppe, welche am besten mit einem Fluoridreagens, wie beispielsweise Tetrabutylammoniumfluorid (TBAF) in THF erfolgt, vorzugsweise bei Raumtemperatur, wird die freiwerdende Hydroxylgruppe zum Aldehyd der allgemeinen Formel (XIII), 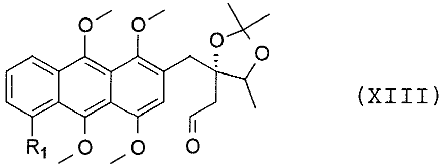 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; oxidiert, vorzugsweise mit einer Swern Oxidation.

Die so erhaltene Verbindung der allgemeinen Formel (XIII) durch oxidative Dealkylierung bevorzugt unter Anwendung eines Cersalzes (Cer(IV)-ammoniumnitrat, vorzugsweise in Acetonitril bei 0°C bis 10°C, am meisten bevorzugt bei ungefähr 2°C) zum Anthrachinon-Derivat der allgemeinen Formel (XIV), 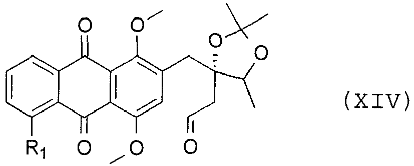 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; umgesetzt.

Die restlichen Schutzgruppen (z.B. Methoxy und Acetonid) werden unter sauren Bedingungen abgespalten, wobei zu diesem Zweck vorzugsweise Lewis-Säuren wie BBr₃ (Bortribromid), BI₃ (Bortriiodid), BF₃ (Bortrifluorid) oder AlCl₃ (Aluminiumchlorid), vorzugsweise BCl₃ (Bortrichlorid) in DCM, vorzugsweise bei 0°C-10°C, am meisten bevorzugt bei ungefähr 2°C zum Einsatz kommen, wobei das Halbacetal der allgemeinen Formel (XV), 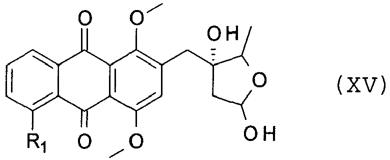 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; erhalten wird.

Formel (XV) wird anschließend zyklisiert, vorzugsweise mit Hilfe einer Marschalk Reaktion in Tetrahydrofuran/Methanol als Lösungsmittelin, vorzugsweise bei einem Temperaturbereich von - 10°C bis Raumtemperatur, zur Verbindung der allgemeinen Formel (XVI) 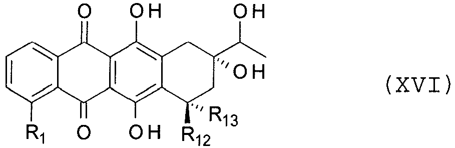 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₁₂ und R₁₃ jeweils für ein Wasserstoffatom, oder ein Wasserstoffatom und eine Hydroxylgruppe in beliebiger Kombination stehen.

Die Seitenketten-Hydroxylgruppe wird anschießend oxidiert, wobei sich für diesen Zweck besonders Dess-Martin Periodinan in Dichlormethan eignet, vorzugsweise bei Raumtemperatur, um so eine Verbindung der allgemeinen Formel (XVII), 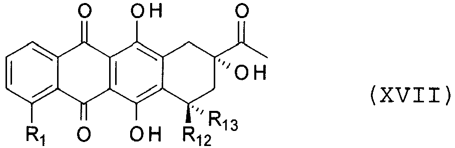 in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; R₁₂ und R₁₃ jeweils für ein Wasserstoffatom, oder ein Wasserstoffatom und eine Hydroxylgruppe in beliebiger Kombination stehen; zu erhalten.

Die Verbindung der Formel (XVII) wird anschließend, vorausgesetzt R₁₂ und R₁₃ stehen für ein Wasserstoffatom, hydroxyliert, wobei sich dafür besonders N-Bromsuccinimid und Azo-bis-(isobutyronitril) in CCl₄ eignen; vorzugsweise erfolgt die Hydroxylierung unter Rückfluß, wobei eine Verbindung der allgemeinen Formel (IV), in welcher in welcher R₁ ein Wasserstoffatom, eine Hydroxy- oder Methoxy-Gruppe, ein Halogenatom, insbesondere ein Fluor-, Chlor- oder Bromatom, oder eine NO₂-Gruppe ist; und R₂ Wasserstoff bedeutet, entsteht. 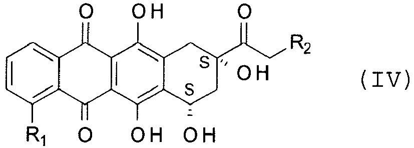 Verbindungen gemäß der allgemeinen Formel (I), (Ia) und (IIIc) der vorliegenden Erfindung zeigen in nichtradioaktiven Zellproliferations- und Zytotoxizitätstests an Tumorzelllinien, beispielsweise den beiden Tumorzelllinien MCF-7 und KB-3-1, statistisch signifikante Effekte bezüglich ihrer cytotoxischen Wirkung und ihrer Eigenschaft die Zellproliferation zu hemmen.

Demzufolge betrifft die vorliegende Erfindung des Weiteren pharmazeutische Zusammensetzung umfassend eine oder mehrere Verbindungen der Formel (I), (Ia) oder (IIIc) und optional einen oder mehrere pharmazeutisch annehmbare Stoffe, ausgewählt aus Hilfsstoffen, Träger, Verdünnungsmittel und Lösungsmitteln.

Geeignete Hilfsstoffen, Träger, Verdünnungsmittel und Lösungsmitteln sind dem Fachmann bekannt.

Die vorliegende Erfindung betrifft weiterhin einen pharmazeutischen Kit umfassend (i) eine oder mehrere Verbindungen der Formel (I), (Ia) oder (IIIc), oder eine pharmazeutische Zusammensetzung umfassend eine oder mehrere Verbindungen der Formel (I), (Ia) oder (IIIc) und optional einen oder mehrere pharmazeutisch annehmbare Stoffe, ausgewählt aus Hilfsstoffen, Träger, Verdünnungsmittel und Lösungsmitteln und (ii) mindestens einem antiproliferativen oder cytotoxischen Wirkstoff.

Die Verbindungen der Formel (I), (Ia) oder (IIIc), eine pharmazeutische Zusammensetzung oder ein pharmazeutischer Kit gemäß der vorliegenden Erfindung können als Medikament oder als Werkzeug in der biomedizinischen Forschung verwendet werden. Verbindungen der Formel (I), (Ia) oder (IIIc), eine pharmazeutische Zusammensetzung oder ein pharmazeutischer Kit gemäß der vorliegenden Erfindung können darüber hinaus zur Anwendung bei der

Behandlung von Krankheiten oder Krankheitszuständen, die zumindest teilweise durch Therapie gelindert werden können, verwendet werden. Die Krankheiten oder Krankheitszustände sind ausgewählt aus proliferierenden Krankheiten, vorzugsweise Krebs.

Die Verbindungen der Formel (I), (Ia) und (IIIc), eine pharmazeutische Zusammensetzung oder ein pharmazeutischer Kit gemäß der vorliegenden Erfindung können als Monopräparate oder in Kombination mit anderen Wirkstoffen, vorzugsweise Wirkstoffen, welche ebenfalls die Zellproliferation hemmen, bzw. eine zytotoxische Wirkung entfalten können, eingesetzt werden. Beispielhaft seien die bekannten Wirkstoffe Bleomycin, Vinblastin, Dacarbazin, Cyclophosphamid, Etoposid(phosphat), Procarbazin, Vincristin, Prednison, Cisplatin, Carboplatin, 5-Fluoruracil, Docetaxel, erwähnt, welche schon jetzt mit Anthracyclinen in Kombination eingesetzt werden (z.B. ABVD, BEACOPP, ECF, TAC, TEC).

Ebenfalls können die Verbindungen der Formel (I), (Ia) und (IIIc) der vorliegenden Erfindung zusammen mit rekombinanten Wirkstoffen (Biologika) verwendet werden, welche die Wirkung der erfindungsgemäßen Verbindungen spezifisch, z.B. als Antikörper, verstärken können.

Die vorliegende Erfindung wird nun anhand nachfolgender Beispiele näher erläutert.

### Beispiel 1:

### N-[(2S,3S,4S)-3,6-dihydroxy-2-[2-[2-[2-[2-(2-methoxyethoxy)ethoxy]ethoxy]ethoxy]ethoxymethyl]tetrahydropyran-4-yl]-2,2,2-trifluoro-acetamide; Formel (III); R₄=[(-CH₂-CH₂-O)₅-CH₃] ; X=O; Y=[CH-NR₅R₆], R₅=H, R₆=[C(=O)CF₃]

Zu einer Lösung von 8,9 g (17.2 mmol) N-[2-(1,3-dioxolan-2-yl)-1-[(4S,5S)-5-[2-[2-[2-[2-(2-methoxyethoxy)ethoxy]ethoxy] ethoxy]ethoxymethyl]-2,2-dimethyl-1,3-dioxolan-4-yl]ethyl]-2,2,2-trifluoro-acetamide (II) in THF/H₂0 (4:1) werden 43 ml (0.56 mol) Trifluoressigsäure zugetropft. Anschließend wird bei 60°C für 30 min gerührt. Dann wird das Reaktionsgemisch auf 100 ml Eiswasser geleert und 87 g (1.03 mol) festes NaHCO₃ zugesetzt, bis pH 6-7 erreicht ist. Das Reaktionsgemisch wird anschließend filtriert und mit 3x mit je 200 ml Dichlormethan extrahiert. Die vereinigten organischen Phasen werden über Natriumsulfat getrocknet, filtriert und eingedampft. Das erhaltene Öl wird chromatographisch gereinigt (Laufmittel DCM/MeOH 100:3-> 100:5-> 10:1, v/v) Es werden 2,1 g (27%) farbloses Öl isoliert. Das Anomerenverhältnis beträgt α:β= 76:24.
¹H NMR (α), (500 MHz; CDCl₃): δ(ppm)= 7,09 (m, 1H, NH); 5,41 (m, 1H, H-1); 4,47 (m, 1H, H-3); 4,18 (m, 1H, H-5); 3,92 (m, 1H, H-4); 3,79-3,69 (m, 2H, H-6/1, H-6/2) 3,62 (m, 18H, CH₂O); 3,55 (m, 2H, MeOCH₂); 3,35 (s, 3H, OMe); 2,00-1,91 (m, 1H, H-2/1); 1,87-1,84 (m, 1H, H-2/2)
¹H NMR (β), (500 MHz; CDCl₃) : δ(ppm)= 7,29 (m, 1H, NH); 5,18 (m, 1H, H-1); 4,16-4,06 (m, 1H, H-3); 3,82 (m, 1H, H-4); 3,79-3,69 (m, 2H, H-6/1, H-6/2); 3,62 (m, 19H, CH₂O, H-5); 3,55 (m, 2H, CH₂O); 3,35 (s, 3H, OMe); 2,04-2,00 (m, 1H, H-2/1); 1,80-1,74 (m, 1H, H-2/2)

### Beispiel 2:

### [(4S,5S,6S)-6-[2-[2-[2-[2-(2-methoxyethoxy)ethoxy]ethoxy] ethoxy]ethoxymethyl] -5-(4-nitrobenzoyl)oxy-4- [(2,2,2-trifluoroacetyl)amino]tetrahydropyran-2-yl] 4-nitrobenzoate; Formel (IIIa) ; R₃=R₁₁=[C(=O)PhNO₂]; R₄=[(-CH₂-CH₂-O)₅-CH₃] ; X= O; Y=[CH-NR₅R₆], R₅=H, R₆=[C(=O)CF₃]

Zu einer Lösung von 1,67g (3.38 mmol) N-[(3S,4S,6S)-3,6-dihydroxy-2-[2-[2-[2-[2-(2-methoxyethoxy)ethoxy]ethoxy]ethoxy] ethoxymethyl]tetrahydropyran-4-yl] -2,2,2-trifluoro-acetamide (II) in Pyridin wird unter Inertgas auf 0°C abgekühlt. Anschließend werden 1,75 g (9.43 mmol) p-Nitrobenzoylchlorid zugegeben und das Reaktionsgemisch innerhalb 12 h auf Raumtemperatur erwärmt. Die Reaktion wird mit H₂O gequencht und anschließend werden alle Lösungsmittel abgedampft. Der Rückstand wird in Dichlormethan aufgenommen und einmal H₂O, dreimal mit halbgesättigter NaHCO₃-Lösung und einmal mit gesättigter NaCl-Lösung gewaschen, über Natriumsulfat getrocknet, filtriert und eingedampft. Das erhaltene Öl wird chromatographisch gereinigt (Laufmittel Ethylacetat/Petrolether 1:1-> 3:2-> 2:1, v/v) Es werden 1,94 g (72%) als weißes Schaumharz erhalten. Das Anomerenverhältnis beträgt α:β= 76:24.
¹H NMR (α), (200 MHz; CDCl₃) : δ (ppm) = 8,30 (m, 8H, Ar); 6,88 (d, 1H, J= 6.56 Hz, NH); 6,67 (m, 1H, H-1); 5,76 (m, 1H, H-4); 4,80 (m, 1H, H-5); 4,46 (m, 1H, H-3); 3,63-3,46 (m, 22H, H-6/1, H-6/2, CH₂O) 3,34 (s, 3H, OMe); 2,39-2,33 (m, 2H, H-2/1, H-2/2)
¹H NMR (β), (200 MHz; CDCl₃) : δ(ppm)= 8, 28 (m, 8H, Ar) ; 6,90 (m, 1H, NH); 6,15 (m, 1H, H-1); 5,64 (m, 1H, H-4); 4,51 (m, 1H, H-5); 4,18(m, 1H, H-3); 3,69-3,52 (m, 22H, H-6/1, H-6/2, CH₂O) 3,34 (s, 3H, OMe); 2,44-2,17 (m, 2H, H-2/1, H-2/2)

### Beispiel 3:

### [(2S,3S,4S,6R)-6-[[(1S,3S)-3-acetyl-3,5,12-trihydroxy-10-methoxy-6,11-dioxo-2,4-dihydro-1H-tetracen-1-yl]oxy]-2-[2-[2-[2-[2-(2-methoxyethoxy)ethoxy]ethoxy]ethoxy]ethoxymethyl]-4-[(2,2,2-trifluoroacetyl)amino]tetrahydropyran-3-yl] 4-nitrobenzoate; Formel (I); R₁=OMe; R₂=H; R₃=[C(=O)PhNO₂]; R₄= [(-CH₂-CH₂-O)₅-CH₃] ; X=O; Y=[CH-NR₅R₆], R₅=H, R₆=[C(=O)CF₃]

Zu einer Lösung von 225 mg (0.28 mmol) [(4S,5S,6S)-6-[2-[2-[2-[2-(2-methoxyethoxy)ethoxy]ethoxy]ethoxy]ethoxymethyl]-5-(4-nitrobenzoyl)oxy-4-[(2,2,2-trifluoroacetyl)amino]tetrahydropyran -2-yl] 4-nitrobenzoate, (III), in 15 ml Dichlormethan und 12 ml Et₂O werden 1,1g Molsieb-4A zugesetzt. Unter Inertgas werden 129 mg (0.58 mmol) Trimethylsilyltrifluoromethanesulfonate bei -40°C zugetropft und 1h bei 0°C gerührt, auf -20°C abgekühlt und 56 mg (0.14 mmol) (7S,9S)-9-acetyl-6,7,9,11-tetrahydroxy-4-methoxy-8,10-dihydro-7H-tetracene-5,12-dione, (IV), gelöst in 9 ml Tetrahydrofuran zugetropft. Anschließend wird 6h bei -10°C bis -15°C gerührt. Dem Reaktionsgemisch werden gesättigte NaHCO₃-Lösung und Dichlormethan zugesetzt und die Phasen getrennt. Die wässrige Phase wir mehrmals mit Dichlormethan extrahiert. Die vereinigten organischen Phasen werden mit H₂O und gesättigter NaCl-Lösung gewaschen, über Natriumsulfat getrocknet, filtriert und eingedampft. Der erhaltene Rückstand wird chromatographisch gereinigt (Laufmittel DCM/MeOH 100:1-> 100:2-> 100:4, v/v). Es werden 118 mg (82%) orange-roter Feststoff erhalten.
¹H NMR (500MHz, CDCl₃): δ(ppm)= 13,93 (s, 1H, OH-6); 13,15 (s, 1H, OH-11); 8,30-8,23 (m, 4H, Ar); 7,90 (d, 1H, J= 7.55 Hz, H-1); 7,76-7,72 (m, 1H, H-2); 7,35 (d, 1H, J= 8.5 Hz, H-3); 6,72 (m, 1H, NH); 5,69 (m, 1H, H-1'); 5,62 (m, 1H, H-4'); 5,20 (m, 1H, H-7); 4,56-4,53 (m, 1H, H-5'); 4,48-4,40 (m, 1H, H-3'); 4,04 (s, 3H, ArOMe); 3,68-3,45 (m, 22H, CH₂O, H-6); 3,33 (s, 3H, OMe); 3,12 (m, 1H, H-10/1); 2,88 (m, 1H, H-10/2); 2,57 (m, 1H, H-8/1); 2,43 (s, 3H, H-14); 2,18-2,02 (m, 3H, H-8/2, H-2')

### Beispiel 4:

### N-[(2S,3S,4S,6R)-6-[[(1S,3S)-3-acetyl-3,5,12-trihydroxy-10-methoxy-6,11-dioxo-2,4-dihydro-1H-tetracen-1-yl]oxy]-3-hydroxy-2-[2-[2-[2-[2-(2-methoxyethoxy)ethoxy]ethoxy]ethoxy] ethoxymethyl]tetrahydropyran-4-yl]-2,2,2-trifluoro-acetamide; Formel (I); R₁=OMe; R₂=R₃=H; R₄=[(-CH₂-CH₂-O)₅-CH₃] ; X=O; Y= [CH-NR₅R₆], R₅=H, R₆=[C(=O)CF₃]

Eine Lösung von 99mg (0.1 mmol) [(2S,3S,4S,6R)-6-[[(1S,3S)-3-acetyl-3,5,12-trihydroxy-10-methoxy-6,11-dioxo-2,4-dihydro-1H-tetracen-1-yl]oxy]-2-[2-[2-[2-[2-(2-methoxyethoxy)ethoxy]ethoxy]ethoxy]ethoxymethyl]-4-[(2,2,2-trifluoroacetyl)amino]tetrahydropyran-3-yl] 4-nitrobenzoate, (I), in 52,1 ml Methanol und 0,1 ml Dichlormethan wird auf 0°C abgekühlt. Anschließend werden 1,3 ml 0.1 N NaOH zugetropft und für 30 min bei 0°C gerührt. Das Reaktionsgemisch wir anschließend mit Eisessig neutralisiert und Ethylacetat und gesättigte NaCl-Lösung zugegeben. Die Phasen werden getrennt und die wässrige Phase wird einmal mit Ehtylacetat extrahiert. Die vereinigten organischen Phasen werden mit gesättigter NaCl-Lösung gewaschen, über Natriumsulfat getrocknet, filtriert und eingedampft. Der erhaltene Rückstand wird chromatographisch gereinigt (Laufmittel DCM/MeOH 100:4, v/v). Es werden 75,9 mg (89%) orange-roter Feststoff erhalten.
¹H NMR (500MHz, CDCl₃) : δ(ppm) = 13,94 (s, 1H, OH-6); 13,23 (s, 1H, OH-11); 7,99 (d, 1H, J= 7.55 Hz, H-1); 7,75 (t, 1H, J= 8.02 Hz H-2); 7,36 (d, 1H, J= 8.5 Hz, H-3); 7,10 (m, 1H, NH); 5,56 (m, 1H, H-1'); 5,25 (m, 1H, H-7); 4,22-4,14 (m, 1H, H-3'); 4,14-4,11 (m, 1H, H-5'); 4,05 (s, 3H, ArOMe); 3,98 (m, 1H, H-4'); 3,87-3,71 (m, 2H, H-6/1, H-6/2); 3,69-3,53 (m, 20H, CH₂O); 3,35 (s, 3H, OMe); 3,27-3,17 (m, 1H, H-10/1); 2,87-2,84 (m, 1H, H-10/2); 2,39 (s, 3H, H-14); 2,37-2,34 (m, 1H, H-8/1); 2,12-2,01 (m, 2H, H-8/2, H-2'/1); 1,86-1,83 (m, 1H, H-2'/2)

### Beispiel 5:

### (7S,9S)-9-acetyl-7-[(2R,4S,5S,6S)-4-amino-5-hydroxy-6-[2-[2-[2-[2-(2-methoxyethoxy)ethoxy]ethoxy]ethoxy]ethoxymethyl] tetrahydropyran-2-yl]oxy-6,9,11-trihydroxy-4-methoxy-8,10-dihydro-7H-tetracene-5,12-dione; Formel (I); R₁=OMe; R₂=R₃=H; R₄=[(-CH₂-CH₂-O)₅-CH₃] ; X=O; Y=[CH-NH₂]

Eine Lösung von 60mg (0.07 mmol) N-[(2S,3S,4S,6R)-6-[[(1S,3S)-3-acetyl-3,5,12-trihydroxy-10-methoxy-6,11-dioxo-2,4-dihydro-1H-tetracen-1-yl]oxy]-3-hydroxy-2-[2-[2-[2-[2-(2-methoxyethoxy)ethoxy]ethoxy]ethoxy]ethoxymethyl]tetrahydropyran-4-yl]-2,2,2-trifluoro-acetamide, (I), in 12,5 ml 1 N NaOH wird bei Raumtemperatur 20 min gerührt. Das Reaktionsgemisch wird anschließend mit 12,5 ml 1N HCL neutralisiert und so lange mit Dichlormethan extrahiert bis die Extrakte keine orange Farbe mehr aufweisen. Die vereinigten organischen Phasen werden mit gesättigter NaCl-Lösung gewaschen, über Natriumsulfat getrocknet, filtriert und eingedampft. Der erhaltene Rückstand wird chromatographisch gereinigt (Laufmittel DCM/MeOH 10:1-> 10:2, v/v). Es werden 13 mg (24%) orange-roter Feststoff erhalten.
¹H NMR (500MHz, CDCl₃): δ(ppm)= 13,81 (s, 1H, OH-6); 13,12 (s, 1H, OH-11); 7,85 (d, 1H, J= 6,9 Hz, H-1); 7,67 (t, 1H, J= 7,57 Hz, H-2); 7,36 (m, 1H, H-3); 5,52 (m, 1H, H-1'); 5,00 (m, 1H, H-7); 4,27 (m, 1H, H-4'); 4,19 (m, 1H, H-5'); 3,95 (s, 3H, ArOMe); 3,91-3,50 (m, 23H, H-3', H-6', CH₂O); 3,33 (s, 3H, OMe); 3,13-3,10 (m, 1H, H-10/1); 2,81-2,77 (m, 1H, H-10/2); 2,38 (s, 3H, H-14); 2,35 (m, 1H, H-8/1); 2,18-2,00 (m, 3H, H-2', H-8/2)

### Beispiel 6:

### [(4S,5S)-5-[2-(1,3-dioxolan-2-yl)-1-hydroxy-ethyl]-2,2-dimethyl-1,3-dioxolan-4-yl]methyl benzoate; Formel (VII) ; R₇=R₈=CH₂; R₉= R₁₀=CH₃; X=O; Y=[CH-OH]

Eine Lösung von 4,20 g (0,17 mol) Magnesiumspäne und 2 mg (0.015 mmol) elementarem Iod in 200 ml THF wird unter Argon für 10 min zum sieden erhitzt. Anschließend werden 26 g (0.15 mol) 2-Bromomethyl-1,3-Dioxolane (V) zugetropft, bis das Anspringen der Reaktion erkennbar wird. Das restliche 2-Bromomethyl-1,3-Dioxolane (V) wird im Anschluss so zu dosiert, dass das Reaktionsgemisch gelinde siedet. Nach beendeter Zugabe wird das Reaktionsgemisch bei 80 °C für 2h gerührt. Im Anschluss werden 20 g (0,08 mol) [(4S,5R)-5-formyl-2,2-dimethyl-1,3-dioxolan-4-yl]methyl benzoate (VI), gelöst in 30ml THF, zum Reaktionsgemisch zugetropft und für 6 h bei Raumtemperatur gerührt. Dem Reaktionsgemisch werden 200 ml gesättigte NH₄Cl-Lösung und 200 ml Eis zugesetzt und für 5 min. gerührt. Anschließend werden 300 ml Ethylacetat zugesetzt, Phasen getrennt und die wässrige Phase mit 100 ml Ethylacetat extrahiert. Die vereinigten organischen Phasen werden mit 100 ml gesättigte NH₄Cl-Lösung gewaschen, über Na₂SO₄ getrocknet, filtriert und eingedampft. Es werden 25 g (92%) gelbes Öl (Mischung der Diastereomere 51:49 an 3') erhalten.
¹H NMR (Diastereomer 1), (500MHz, CDCl₃): δ(ppm)= 8,06(m, 2H, Ar); 7,56 (m, 1H, Ar); 7,44 (m, 2H, Ar); 5,09 (m, 1H, H-1); 4,66 (dd, J1= 11.6 Hz, J2= 2.5 Hz, 1H, H-6/1); 4,39 (dd, J1= 11.6 Hz, J2= 5.6 Hz, 1H, H-6/2); 4,31 (m, 1H, H-5); 4,08-3,80 (m, 6H, H-4, H-3, 2x OCH₂); 2,18-2,14 (m, 1H, H-2/1); 1,90-1,81 (m, 1H, H-2/2); 1,42 (s, 3H, MeC); 1,41 (s, 3H, MeC)
¹H NMR (Diastereomer 2), (500MHz, CDCl₃) : δ(ppm) = 8,06(m, 2H, Ar) ; 7,56 (m, 1H, Ar); 7,44 (m, 2H, Ar); 5,09 (m, 1H, H-1); 4,55 (m, 1H, H-6/1); 4,48-4,31 (m, 2H, H-6/2, H-5); 4,08-3,80 (m, 6H, H-4, H-3, 2x OCH₂) ; 2,03-1,92 (m, 2H, H-2/1, H-2/2); 1,45 (s, 3H, MeC); 1,44 (s, 3H, MeC)

### Beispiel 7:

### [(4S,5R)-5-[2-(1,3-dioxolan-2-yl)acetyl]-2,2-dimethyl-1,3-dioxolan-4-yl]methyl benzoate; Formel (VII); R₇=R₈=CH₂; R₉=R₁₀= CH₃; X=O; Y=[C(=O)]

Eine Lösung von 22,7 ml (0.31 mol) DMSO und 10 ml Dichlormethan wird auf -70°C abgekühlt und unter Inertgas langsam 18,53 g (0,14 mol) Oxalyldichlorid zugetropft, so dass die Temperatur -60°C nicht übersteigt. Das Reaktionsgemisch wird bei -70°C für 50 Minuten gerührt. Anschließend werden 34,22 g (0.1 mol) [(4S,5S)-5-[2-(1,3-dioxolan-2-yl)-1-hydroxy-ethyl]-2,2-dimethyl-1,3-dioxolan-4-yl]methyl benzoate (II) gelöst in 20 ml Dichlormethan zugetropft, wobei zu berücksichtigen ist, dass die Temperatur -60°C nicht übersteigt. Anschließend wird das Reaktionsgemisch 50 min bei -70°C gerührt. Danach werden 79,84 g (0.73 mol) Triethylamin zugetropft und weitere 30 min bei -70°C gerührt. Das Reaktionsgemisch wird dann langsam auf Raumtemperatur erwärmt und 250 ml H₂O und 200 ml Dichlormethan zugesetzt. Die Phasen werden getrennt und die wässrige Phase einmal mit 100 ml Dichlormethan extrahiert. Die vereinigten organischen Phasen werden einmal mit 100 ml Schwefelsäure (0,1 % in H₂O), einmal mit 100 ml gesättigter NaHCO₃-Lösung (und einmal mit 100 ml gesättigter NaCl-Lösung gewaschen über Na₂SO₄ getrocknet, filtriert und eingedampft. Es werden 33,73 g (99%) farbloses Öl erhalten.
¹H NMR (500 MHz; CDCl₃): δ(ppm)= 8,02 (d, J= 7.2 Hz, 2H, Ar); 7,53 (t, J= 7.2 Hz, 1H, Ar); 7,41 (t, J= 7.6 Hz, 2H, Ar); 5,30 (t, J= 5.2 Hz, 1H, H-1); 4,62 (dd, J1= 11.6 Hz, J2= 3.1 Hz, 1H, H-6/1); 4,37 (m, 3H, H-4, H-5, H-6/2); 3,94 (m, 2H, OCH₂); 3,83 (m, 2H, OCH₂) ; 3,04 (d, J= 5.3, 2H, H-2/1, H-2/2); 1,44 (s, 3H, MeC); 1,41 (s, 3H, MeC)

### Beispiel 8:

### [(4S,5S)-5-[(E)-C-(1,3-dioxolan-2-ylmethyl)-N-hydroxy-carbonimidoyl]-2,2-dimethyl-1,3-dioxolan-4-yl]methyl benzoate; Formel (VII); R₇ = R₈= CH₂; R₉ = R₁₀ = CH₃; X= O; Y= [C(=N) -OH]

Zu einer Lösung von 24,52 g (0.07 mol) [(4S,5R)-5-[2-(1,3-dioxolan-2-yl)acetyl]-2,2-dimethyl-1,3-dioxolan-4-yl]methyl benzoate (II) in 80 ml a Pyridin, werden 31,58 g (0.45 mol) Hydroxylaminhydrochlorid (NH₂OH·HCl) zugesetzt. Anschließend wird für 13h bei 55°C unter Inertgas gerührt. Das Pyridin wird mittels Rotationsverdampfer entfernt und der Rückstand anschließend im Hochvakuum getrocknet. Danach werden 250 ml H₂O und 250 ml Ethylacetat zugesetzt und die Phasen getrennt. Die wässrige Phase wird viermal mit Ethylacetat (4x50 ml) extrahiert. Die vereinigten organischen Phasen werden mit gesättigter NaCl-Lösung gewaschen und über Natriumsulfat getrocknet, filtriert und eingedampft. Es werden 24,24 g (94%) gelbes Öl erhalten.
¹H NMR (500 MHz; CDCl₃): δ(ppm)= 8,05 (d, J= 7.2 Hz, 2H, Ar); 7,56 (t, J= 7.2 Hz, 1H, Ar); 7,43 (t, J= 7.7 Hz, 2H, Ar); 5,31 (t, J= 4.7 Hz, 1H, H-1); 4,58 (m, 3H, H-4,H-5,H-6/1); 4,40 (m, 1H, H-6/2); 3,98 (m, 2H, OCH₂); 3,84 (m, 2H, OCH₂) ; 2,78 (m, 2H, H-2/1, H-2/2); 1,46 (s, 6H, Me₂C)

### Beispiel 9:

### 2-(1,3-dioxolan-2-yl)-1-[(4S,5S)-5-(hydroxymethyl)-2,2-dimethyl-1,3-dioxolan-4-yl]ethanone oxime; Formel (II) ; R₇=R₈=CH₂; R₉= R₁₀=CH₃; R₄=H; X=O; Y=[C(=N)-OH]

Zu einer Lösung von 16,0 g (43.79 mmol) [(4S,5S)-5-[(E)-C-(1,3-dioxolan-2-ylmethyl)-N-hydroxy-carbonimidoyl]-2,2-dimethyl-1,3-dioxolan-4-yl]methyl benzoate (II) in 340 ml THF/H₂O (1:1) werden 32ml 2N NaOH (63.96 mmol) zugetropft und für 18 h bei 60°C gerührt. Nach dem abkühlen auf Raumtemperatur werden 200 ml MTBE zugesetzt, die Phasen getrennt und die wässrige Phase viermal mit MTBE extrahiert. Die vereinigten organischen Phasen werden mit gesättigter NaCl-Lösung gewaschen und über Natriumsulfat getrocknet, filtriert und eingedampft. Es werden 9,31 g (81%) gelbes Öl erhalten.
¹H NMR (500 MHz; CDCl₃): δ(ppm)= 9,07 (s, 1H, OH); 5,35 (t, J= 5.3 Hz, 1H, H-1); 4,45 (d, J= 8.5 Hz, 1H, H-4,); 4,27 (m, 1H, H-5); 3,97 (m, 2H, OCH₂) ; 3,78 (m, 3H, OCH₂, H-6); 3,09 (s, 1H, OH); 2,74 (m, 2H, H-2/1, H-2/2); 1,43 (s, 6H, Me₂C)

### Beispiel 10:

### 2-(1,3-dioxolan-2-yl)-1-[(4S,5S)-5-[2-[2-[2-[2-(2-methoxyethoxy)ethoxy]ethoxy]ethoxy]ethoxymethyl] -2,2-dimethyl-1,3-dioxolan-4-yl]ethanone oxime; Formel (II); R₇= R₈= CH₂; R₉ = R₁₀=CH₃; R₄=[(-CH₂-CH₂-O)₅-CH₃] ; X=O; Y=[C(=N)-OH]

Zu einer Lösung von 7.8 g (29.9 mmol) 2-(1,3-dioxolan-2-yl)-1-[(4S,5S)-5-(hydroxymethyl)-2,2-dimethyl-1,3-dioxolan-4-yl]ethanone oxime (II) in 150 ml THF werden 2,26 g Natriumhydrid (95%) (89.8 mmol) portionsweise zugesetzt und 1,5h unter Inertgas bei Raumtemperatur gerührt. Anschließend werden 24.3 g (60 mmol) 2-[2-[2-[2-(2-methoxyethoxy)ethoxy]ethoxy]ethoxy] ethyl4-methylbenzenesulfonate gelöst in 50 ml THF zugetropft und für weitere 5h bei 60°C gerührt. Die Reaktion wird im Anschluss mit gesättigter NH₄Cl-Lösung gequencht. Danach werden 300 ml DCM zugegeben, die Phasen getrennt und die wässrige Phase viermal mit DCM extrahiert. Die vereinigten organischen Phasen werden über Na₂SO₄ getrocknet, filtriert und eingedampft. Das erhaltene Öl wird chromatographisch gereinigt (Laufmittel Toluol/Aceton 2:1-> 1:1, v/v). Es werden 12,4 g (84%) gelbes Öl erhalten.
¹H NMR (500 MHz; CDCl₃) : δ(ppm)= 5,32 (t, J= 5.3 Hz, 1H, H-1); 4,38 (m, 1H, H-5); 4,28 (d, J= 8.2 Hz, 1H, H-4,); 3,95 (m, 2H, OCH₂) ; 3,82 (m, 2H, OCH₂) ; 3,64 (m, 18H, CH₂O) ; 3,52 (m, 4H, H-6, MeOCH₂) ; 3,35 (s, 3H, OMe); 2,73 (d, J= 5.7 Hz, 2H, H-2/1, H-2/2); 1,42 (s, 3H, MeC); 1,40 (s, 3H, MeC)

### Beispiel 11:

### (1S)-2-(1,3-dioxolan-2-yl)-1-[(4S,5S)-5-[2-[2-[2-[2-(2-methoxyethoxy)ethoxy]ethoxy]ethoxy]ethoxymethyl]-2,2-dimethyl-1,3-dioxolan-4-yl]ethanamine; Formel (II); R₇=R₈=CH₂; R₉=R₁₀= CH₃; R₄=[(-CH₂-CH₂-O)₅-CH₃]; X=O; Y=[CH-NH₂] ; Y=[CH-NR₅R₆], R₅= R₆= H

Eine Lösung von 12,4 g (25 mmol) 2-(1,3-dioxolan-2-yl)-1-[(4S,5S)-5-[2-[2-[2-[2-(2-methoxyethoxy)ethoxy]ethoxy]ethoxy]ethoxymethyl]-2,2-dimethyl-1,3-dioxolan-4-yl]ethanone oxime (II) in 125 ml THF wird unter Intergas auf 0°C abgekühlt. Anschließen werden 3,1 g (82.2 mmol) Lithiumaluminiumhydrid (LiAlH₄) unter Rühren langsam zugesetzt. Anschließend wird 4h bei RT gerührt. Die Reaktion wird mit NaOH-Lösung (5M) gequencht, 100 ml EtOAc zugesetzt und für 10 min gerührt. Das Reaktionsgemisch wird im Anschluss filtriert und die Phasen getrennt. Die vereinigten organischen Phasen werden mit gesättigter NaCl-Lösung gewaschen und über Natriumsulfat getrocknet, filtriert und eingedampft. Es werden 9,8 g (81%) gelbes Öl (Mischung der Diastereomere 57:43 an 3') erhalten.
¹H NMR, (200 MHz; CDCl₃) : δ(ppm)= 5,01 (t, J= 4.73 Hz, 1H, H-1); 4,20-3,77 (m, 7H, , H-4, H-5, H-6, 2x OCH₂); 3,64 (m, 18H, CH₂O); 3,53 (m, 2H, MeOCH₂); 3,35 (s, 3H, OMe); 3,19-3,00 (m, 1H, H-3); 2,24-1,56 (m, 2H, H-2); 1,38 (s, 3H, MeC); 1,37 (s, 3H, MeC)

### Beispiel 12:

### N-[2-(1,3-dioxolan-2-yl)-1-[(4S,5S)-5-[2-[2-[2-[2-(2-methoxyethoxy)ethoxy]ethoxy]ethoxy]ethoxymethyl]-2,2-dimethyl-1,3-dioxolan-4-yl]ethyl]-2,2,2-trifluoro-acetamide; Formel (II); R₇=R₈=CH₂; R₉=R₁₀=CH₃; R₄=[(-CH₂-CH₂-O)₅-CH₃]; X=O; Y=[CH-NR₅R₆], R₅=H, R₆=[C(=O)CF₃]

Zu einer Lösung von 9,8 g (20,3 mmol) (1S)-2-(1,3-dioxolan-2-yl)-1-[(4S,5S)-5-[2-[2-[2-[2-(2-methoxyethoxy)ethoxy]ethoxy]ethoxy]ethoxymethyl]-2,2-dimethyl-1,3-dioxolan-4-yl]ethanamine (II) in 196 ml DCM und 9,6 ml Pyridin werden 0,05 g (0.4 mmol) 4-(Dimethylamino)-pyridin zugesetzt. Das Reaktionsgemisch wird auf -17°C abgekühlt und 6,41 g (30.5 mmol) Trifluoressigsäureanhydrid zugetropft. Im Anschluss wird innerhalb von 4h unter rühren auf Raumtemperatur erwärmt, alle Lösungsmittel eingedampft und der Rückstand in Dichlormethan aufgenommen. Die organische Phase wird mit halbgesättigter NaHCO₃-Lösung und gesättigter NaCl-Lösung gewaschen, über Natriumsulfat getrocknet, filtriert und eingedampft. Das erhaltene Öl wird chromatographisch gereinigt (Laufmittel Petrolether/Aceton 2:1, v/v). Es werden 9,5 g (80%) gelbes Öl (Mischung der Diastereomere 57:43 an 3') erhalten.
¹H NMR (Diastereomer 1), (500 MHz; CDCl₃): δ(ppm)= 7,65 (d, J= 8.85 Hz, 1H, NH); 4,99 (t, J= 4.6 Hz, 1H, H-1); 4,30 (m, 1H, H-3); 3,92 (m, 7H, , H-4, H-5, H-6, 2x OCH₂); 3,62 (m, 18H, CH₂O); 3,52 (m, 2H, MeOCH₂); 3,35 (s, 3H, OMe); 2,04 (m, 2H, H-2); 1,38 (s, 3H, MeC); 1,37 (s, 3H, MeC)
¹H NMR (Diastereomer 2), (500 MHz; CDCl₃): δ(ppm)= 6,95 (d, J= 9.15 Hz, 1H, NH); 4,96 (t, J= 4.4 Hz, 1H, H-1); 4,40 (m, 1H, H-3); 3,92 (m, 7H, , H-4, H-5, H-6, 2x OCH₂); 3,62 (m, 18H, CH₂O) ; 3,52 (m, 2H, MeOCH₂); 3,35 (s, 3H, OMe); 2,04 (m, 2H, H-2); 1,38 (s, 3H, MeC); 1,1,37 (s, 3H, MeC)

### Beispiel 13:

### (2S,5S)-2-tert-butyl-5-[2-(tert-butyl(dimethyl)silyl)oxyethyl]-1,3-dioxolan-4-on; Formel (IX)

40 g *2-[(2S,4S)-2-tert-Butyl-5-oxo-1,3-dioxolan-4-yl]essigsäure* werden unter Argon-atmosphäre in 300 mL THF gelöst und auf 0°C gekühlt. 238 mL BH₃.THF-Komplex (1M in THF) werden innerhalb einer Stunde langsam so zugegeben, dass die Temperatur nicht über 5 °C steigt. Nach der vollständigen Zugabe des Reagens wird das Reaktionsgemisch 20 min bei 0 °C gerührt, auf Raumtemperatur erwärmt und 3,5 Stunden gerührt. Das Reaktionsgemisch wird zwischen gesättigter NH₄Cl-Lösung und EtOAc verteilt. Die Phasen werden getrennt und die wässrige Phase mit EtOAc extrahiert. Die vereinigten organischen Phasen werden mit 5% wässriger NaHCO₃-Lösung und gesättigter Kochsalzlösung gewaschen, über Na₂SO₄ getrocknet, filtriert und im Vakuum eingedampft. Es werden 32 g Zwischenprodukt erhalten, welches ohne weitere Aufreinigung direkt in die nächste Stufe eingesetzt werden kann.

43 g TBDMSCl in 500 mL DCM werden mit 45,1 g Pyridin versetzt. Die Lösung wird 10 min gerührt, dann wird das Zwischenprodukt, gelöst in 100 mL DCM, zugegeben. Das Reaktionsgemisch wird 16 Stunden bei Raumtemperatur gerührt und anschließend auf Wasser gegossen. Die Phasen werden getrennt, die organische Phase wird mit 5% wässriger NaHCO₃-Lösung gewaschen, über Na₂SO₄ getrocknet, filtriert und im Vakuum eingedampft. Das Rohpodukt wird mittels Flash-Chromatographie über Kieselgel mit Toluol als Laufmittel gereinigt. Es werden 52.3 g *(2S, 5S)-2-tert-butyl-5-[2-(tert-butyl(dimethyl)silyl)oxyethyl]-1,3-dioxolan-4-on* als farbloses Öl erhalten.
¹H NMR (500 MHz; CDCl₃) : δ (ppm) = 5.15 (s, 1H, H-5); 4.43 (dd, 1H, 3.8 Hz, 8.5 Hz, H-3); 3.80 (m, 2H, H-1); 2.12 (m, 1H, H-2/1); 1.86 (m, 1H, H-2/2); 0.97 (s, 9H, H-tBuCH₃); 0.89 (s, 9H, H-SitBuCH₃); 0.06 (s, 6H, H-SiCH₃).

### Beispiel 14 (Alkylierungsreaktion):

### (2S,5S)-2-tert-butyl-5-[2-(tert-butyl(dimethyl)silyl)oxyethyl]-5-[(1,4,9,10-tetramethoxy-2-anthryl)methyl]-1,3-dioxolan-4-one; Formel (X); R₁= H

Zu einer Lösung von 18 g KHMDS in 680 mL wasserfreiem THF wird unter Argonatmosphäre bei -76°C eine Lösung von 25,6 g *(2S,5S)-2-tert-Butyl-5-[2-(tert-butyl(dimethyl)silyl)oxyethyl]-1,3-dioxolan-4-on* in 30 mL THF so zugetropft, dass die Temperatur nicht über -72°C steigt. Das Reaktionsgemisch wird 50 Minuten bei -76°C gerührt. Eine Lösung von 22 g *2-(Brommethyl)-1,4,9,10-tetramethoxy-anthracen* in 40 mL THF wird bei -75°C zugetropft. Anschließend wird 20 min bei dieser Temperatur gerührt. Das Reaktionsgemisch wird zwischen 1N HCl und EtOAc verteilt. Die wässrige Phase wird mit EtOAc extrahiert und die vereinigten organischen Phasen werden über Na₂SO₄ getrocknet, filtriert und im Vakuum eingedampft. Das Rohprodukt wird in einer Mischung von 50 mL MTBE und 200 mL PE digeriert, filtriert und mit PE gewaschen. Es werden 23.4 g *(2S,5S)-2-tert-Butyl-5-[2-(tert-butyl(dimethyl)silyl)oxyethyl]-5-[(1,4,9,10-tetramethoxy-2-anthryl)-methyl-1,3-dioxolan-4-on* als gelber Feststoff erhalten, Das Filtrat wird im Vakuum eingedampft und mittels Säulenchromatographie (Kieselgel, Toluol/ EtOAc 30/1) aufgetrennt. Es werden so weitere 7.4 g *(2S,5S)-2-tert-Butyl-5-[2-(tert-butyl(dimethyl)silyl)oxyethyl]-5-[(1,4,9,10-tetra-methoxy -2-anthryl)methyl]-1,3-dioxolan-4-on* erhalten.
¹H NMR (500 MHz; CDCl₃) : δ (ppm) = 8.35 (m, 2H, H-5 and H-8); 7.52 (m, 2H, H-6 and H-7); 6.64 (s, 1H, H-3); 4.84 (s, 1H, H-acetal); 4.02 (s, 3H, OCH₃-4); 4.00 (s, 3H, OCH₃-10); 3.95 (s, 3H, OCH₃-9); 3.83 (m, 2H, H-4'); 3.75 (s, 3H, OCH₃-1); 3.44 (d, 1H, J = 13.9 Hz, H-1'/1); 3.18 (d, 1H, J = 13.9 Hz, H-1'/2); 2.17 (m, 2H, H-3'); 0.89 (s, 9H, H-tBuCH₃); 0.88 (s, 9H, H-SitBuCH₃); 0.05 (s, 3H, H-SiCH₃); 0.04 (s, 3H, H-SiCH₃).

### Beispiel 15:

### (3S)-5-(tert-Butyl(dimethyl)silyl)oxy-3-hydroxy-3-[(1,4,9,10-tetramethoxy-2-anthryl)methyl]pentan-2-on; Formel (XI); R₁= H

Zu einer Lösumg von 62 g *(2S,5S)-2-tert-Butyl-5-[2-(tert-butyl(dimethyl)silyl)oxyethyl]-5-[(1,4,9,10-tetramethoxy-2-anthryl)methyl]-1,3-dioxolan-4-one* in 700 mL wasserfreiem THF werden unter Argonatmosphäre bei -78°C 164 mL MeLi (1.6 M in Et₂O) so zugetropft, dass die Temperatur nicht über -71 °C steigt. Das Reaktionsgemisch wird 1,5 Stunden bei -75°C gerührt und anschließemd zwischen gesättigter NH₄Cl-Lösung und EtOAc verteilt. Die wässrige Phase wird mit EtOAc extrahiert. Die vereinigten organischen Phasen werden über Na₂SO₄ getrocknet, filtriert und im Vakuum eingedampft. Das Rohprodukt wird mittels Säulenchromatographie (Kieselgel, Toluol/EtOAc, 10/1) gereinigt. Es werden 54.2 g *(3S)-5-(tert-Butyl(dimethyl)silyl)oxy-3-hydroxy-3-[(1,4,9,10-tetramethoxy-2-anthryl)methyl]pentan-2-on* als gelber Schaum erhalten.
¹H NMR (500 MHz; CDCl₃): δ(ppm) = 16.67 (m, 2H, H-5 and H-8); 7.51 (m, 2H, H-6 and H-7); 6.70 (s, 1H, H-3); 4.01 (s, 3H, OCH₃-4); 3.98 (s, 3H, OCH₃-10); 3.92 (s, 3H, OCH₃-9); 3.82 (td, 1H, J = 4.1 Hz, 9.2 Hz, H-4'/1); 3.77 (s, 3H, OCH₃-1); 3.70 (dt, 1H, J = 5.1 Hz, 10.4 Hz, H-4'/2); 3.27 (d, 1H, J = 12.9 Hz, H-1'/1); 3.12 (d, 1H, J = 12.9 Hz, H-1'/2); 2.39 (m, 1H, H-3'/1); 2.33 (s, 3H, CH₃); 1.96 (dt, 1H, J = 4.4 Hz, 14.2 Hz, H-3'/2); 0.86 (s, 9H, H-SitBuCH₃); 0.02 (s, 3H, H-SiCH₃); 0.01 (s, 3H, H-SiCH₃).

### Beispiel 16:

### a. Reduktion der Ketogruppe

### (2S,3S)-5-(tert-Butyl(dimethyl)silyl)oxy-3-[(1,4,9,10-tetramethoxy-2-anthryl)methyl]-pentan-2,3-diol.

Zu einer Lösung von 33.8 g *(3S)-5-(tert-Butyl(dimethyl)silyl)oxy-3-hydroxy-3-[(1,4,9,10-tetramethoxy-2-anthryl)methyl]pentan-2-on* in 340 mL EtOH werden unter Argonatmosphäre 2,36 g NaBH₄ zugegeben. Das Reaktionsgemisch wird eine Stunde bei RT gerührt und anschließend mit gesättigter Kochsalzlösung und EtOAc gequencht. Die wässrige Phase wird mit EtOAc extrahiert. Die vereinigten organischen Phasen werden über Na₂SO₄ getrocknet, filtriert und im Vakuum eingedampft. Das Rohprodukt wird mittels Flash- Chromatographie (Kieselgel, Toluol/EtOAc = 5:1) gereinigt. Es werden 30.70 g *(2S,3S)-5-(tert-butyl(dimethyl)silyl)oxy-3-[(1,4,9,10-tetramethoxy-2-anthryl)methyl]pentan-2,3-diol* als gelber Schaum erhalten.

### b. Ketalisierung:

### tert-Butyl-dimethyl-[2-[(4S,5S)-2,2,5-trimethyl-4-[(1,4,9,10-tetramethoxy-2-anthryl)-methyl]-1,3-dioxolan-4-yl]ethoxy]silan; Formel (XII), R₁= H

Zu einer Lösung von 5,26 g des alkoholischen Zwischenproduktes in 100 mL trockenem Aceton werden unter Argonatmosphäre 2,6 mL Dimethoxypropan gefolgt von 0,09 g pTsOH zugegeben. Das Reaktionsgemisch wird 1,5 Stunden bei RT gerührt und anschließend zwischen gesättigter NaHCO₃ - Lösung und EtOAc verteilt. Die Phasen werden getrennt: Die wässrige Phase wird mit EtOAc extrahiert. Die vereinigten organischen Phasen werden über Na₂SO₄ getrocknet, filtriert und im Vakuum eingedampft. Das Rohprodukt wird mittels Flash-Chromatographie (Kieselgel, Toluol / EtOAc, 40/1) gereinigt. Es werden 4.66 g *tert-Butyl-dimethyl-[2-[(4S,5S)-2,2,5-trimethyl-4-[(1,4,9,10-tetramethoxy-2-anthryl)methyl]-1,3-dioxolan-4-yl]ethoxy]silan* als gelber Schaum erhalten.
¹H NMR (500 MHz; CDCl₃): δ(ppm) = 8.35 (m, 2H, H-5 and H-8); 7.50 (m, 2H, H-6 and H-7); 6.97 (s, 1H, H-3); 4.54 (q, 1H, J = 6.3 Hz, H-5'); 4.02 (s, 3H, OCH₃-4); 3.98 (s, 3H, OCH₃-10); 3.92 (s, 3H, OCH₃-9); 3.73 (s, 3H, OCH₃-1); 3.69 (m, 2H, H-4'); 3.19 (d, 1H, J = 13.6 Hz, H-1'/1); 2.75 (d, 1H, J = 13.6 Hz, H-1'/2); 1.91 (m, 1H, H-3'/1); 1.77 (m, 1H, H-3'/2); 1.74 (s, 3H, H-Acetonid); 1.45 (d, 3H, J = 6.3 Hz, CH₃); 1.44 (s, 3H, H-Acetonid); 0.86 (s, 9H, H-SitBuCH₃); 0.01 (s, 6H, H-SiCH₃).

### Beispiel 17:

### a. Abspaltung der TBDMS - Gruppe

### 2-[(4S,5S)-2,2,5-Trimethyl-4-[(1,4,9,10-tetramethoxy-2-anthryl)methyl]-1,3-dioxolan-4-yl]ethanol

Zu eine Lösung von 4.57 g *tert-Butyl-dimethyl-[2-[(4S,5S)-2,2,5-trimethyl-4-[(1,4,9,10-tetramethoxy-2-anthryl)methyl]-1,3-dioxolan-4-yl]ethoxy]silan* in 90 mL wasserfreiem THF werden unter Argonatmosphäre 19,5 mL TBAF (1M in THF) bei RT zugegeben. Die Reaktionsmischung wird 1 Stunde bei RT gerührt. Das Reaktionsgemisch wird zwischen gesättigter NaHCO₃ - Lösung und EtOAc verteilt. Die wässrige Phase wird mit EtOAc extrahiert. Die vereinigten organischen Phasen werden über Na₂SO₄ getrocknet, filtriert und im Vakuum eingedampft. Es werden 603g *2-[(4S,5S)-2,2,5-trimethyl-4-[(1,4,9,10-tetramethoxy-2-anthryl)methyl]-1,3-dioxolan-4-yl]ethanol* als gelber Schaum erhalten.
¹H NMR (500 MHz; CDCl₃): δ(ppm) = 8.35 (m, 2H, H-5 and H-8); 7.51 (m, 2H, H-6 and H-7); 6.93 (s, 1H, H-3); 4.37 (q, 1H, J = 6.3Hz, H-5'); 4.02 (s, 3H, OCH₃-4); 4.00 (s, 3H, OCH₃-10); 3.93 (s, 3H, OCH₃-9); 3.81 (m, 1H, H-4'/1); 3.78 (s, 3H, OCH₃-1); 3.66 (m, 1H, H-4'/2); 3.22 (d, 1H, J = 13.6 Hz, H-1'/1); 2.81 (brs, 1H, OH-4'); 2.75 (d, 1H, J = 13.6 Hz, H-1'/2); 1.88 (m, 2H, H-3'); 1.75 (s, 3H, H-Acetonid); 1.48 (d, 3H, J = 6.3 Hz, CH₃); 1.47 (s, 3H, H-Acetonid) .

### b. Oxidation des primären Alkohols zum Aldehyd:

### 2-[(4S,5S)-2,2,5-Trimethyl-4-[(1,4,9,10-tetramethoxy-2-anthryl)methyl]-1,3-dioxolan-4-yl]acetaldehyd; Formel (XIII); R₁= H

In eine Lösung von 0,99 mL DMSO in 55 mL DCM werden bei -70 °C 0,61 mL Oxalylchlorid unter Argonatmosphäre zugetropft. Das Gemisch wird 1 Stunde bei -70°C gerührt. Anschließend werden 2,19 g *2-[(4S,5S)-2,2,5-trimethyl-4-[(1,4,9,10-tetramethoxy-2-anthryl-)methyl]-1,3-dioxolan-4-yl]ethanol* in 4 mL DCM bei -70°C langsam zugegeben. Das Reaktionsgemisch wird 1 Stunde gerührt. Es werden 4,27 mL Et₃N bei derselben Temperatur zugegeben. Es wird unter langsamer Erwärmung auf 0°C noch 1 Stunde weiter gerührt. Das Reaktionsgemisch wird zwischen gesättigter Kochsalzlösung und EtOAc verteilt. Die organische Phase wird nacheinander mit gesättigter NH₄Cl-Lösung, gesättigter NaHCO₃₋ Lösung und gesättigter Kochsalzlösung gewaschen, über Na₂SO₄ getrocknet, filtriert und im Vakuum eingedampft. Es werden 2.1 g *2-[(4S,5S)-2,2,5-trimethyl-4-[(1,4,9,10-tetramethoxy-2-anthryl)methyl]-1,3-dioxolan-4-yl]acetaldehyd* als gelber Schaum erhalten.
¹H NMR (500 MHz; CDCl₃) : δ (ppm) = 9.65 (dd, 1H, J = 1.3 Hz, 2.2 Hz, H-4'); 8.34 (m, 2H, H-5 und H-8); 7.51 (m, 2H, H-6 und H-7); 6.81 (s, 1H, H-3); 4.28 (q, 1H, J = 6.3 Hz, H-5'); 4.03 (s, 3H, OCH₃-4); 3.99 (s, 3H, OCH₃-10); 3.91 (s, 3H, OCH₃-9); 3.70 (s, 3H, OCH₃-1); 3.23 (d, 1H, J = 13.6 Hz, H-1'/1); 2.77 (dd, 1H, J = 2.2 Hz, 16.4 Hz, H-3'/1); 2.73 (d, 1H, J = 13.6 Hz, H-1'/2); 2.62 (dd, 1H, J = 1.3 Hz, 16.34 Hz, H-3'/2); 1.74 (s, 3H, H-Acetonid); 1.55 (d, 3H, J = 6.3 Hz, CH₃); 1.40 (s, 3H, H-Acetonid).

### c. Oxidative Demethylierung:

### 2-[(4S,5S)-4-[(1,4-dimethoxy-9,10-dioxo-2-anthryl)methyl]-2,2,5-trimethyl-1,3-dioxolan-4-yl]acetaldehyd; Formel (XIV), R₁= H

Zu einer Lösung von 2,08 g 2-[(4S,5S)-2,2,5-trimethyl-4-[(1,4,9,10-tetramethoxy-2-anthryl)-methyl]-1,3-dioxolan-4-yl]acetaldehyd in 60 mL CH₃CN wird bei 2°C einer Lösung von 7,3 g CAN in 130 mL Wasser zugesetzt. Nach 30 min Rühren wird das Reaktionsgemisch mit 80 mL Wasser verdünnt. Die wässrige Phase wird mit EtOAc extrahiert. Die kombinierten organischen Phasen werden getrocknet und eingedampft. Die erhaltene Mischung wird chromatographisch getrennt (Laufmittel Toluol/EtOAc 6:1). Es werden 1.91 g *2-[(4S,5S)-4-[(1,4-dimethoxy-9,10-dioxo-2-anthryl)methyl]-2,2,5-trimethyl-1,3-dioxolan-4-yl]acetaldehyd* als gelber Feststoff erhalten.
¹H NMR (500 MHz; CDCl₃): δ(ppm)= 9.62 (dd, 1H, J = 1.6 Hz, 2.5 Hz, H-4'); 8.16 (m, 2H, H-5 and H-8); 7.71 (m, 2H, H-6 and H-7); 7.45 (s, 1H, H-3); 4.25 (q, 1H, J = 6.3 Hz, H-5'); 4.00 (s, 3H, OCH₃-4); 3.81 (s, 3H, OCH₃-1); 3.18 (d, 1H, J = 12.9 Hz, H-1'/1); 2.66 (d, 1H, J = 12.9 Hz, H-1'/2); 2.65 (dd, 1H, J = 2.5 Hz and 16.4 Hz, H-3'/1); 2.48 (dd, 1H, J = 1.6 Hz, 16.4 Hz, H-3'/2); 1.64 (s, 3H, H-Acetonid); 1.50 (d, 3H, J = 6.3 Hz, CH₃); 1.34 (s, 3H, H-Acetonid).

### Beispiel 18:

### 2-[[(2S,3S)-3,5-dihydroxy-2-methyl-tetrahydrofuran-3-yl]methyl]-1,4-dihydroxy-anthracen-9,10-dion; Formel (XV); R₁= H

Zu einer Lösung von 1 g 2-[(4S,5S)-4-[(1,4-dimethoxy-9,10-dioxo-2-anthryl)methyl]-2,2,5-trimethyl-1,3-dioxolan-4-yl]acetaldehyd in 55 mL DCM werden unter Argonatmosphäre tropfenweise 13,7 mL BCl₃ (1M in DCM) bei 2 °C zugesetzt. Das Reaktionsgemisch wird 40 min gerührt und mit 0.5 N NaOH und DCM versetzt. Die Phasen werden getrennt, die organische Phase wird mit 0.5 N NaOH gewaschen. Die kombinierten wässrigen Phasen werden bei 0°C mit 1 N HCl auf pH = 6 angesäuert und mit DCM extrahiert. Die kombinierten organischen Phasen werden getrocknet und eingedampft. Es werden 0.79 g 2-[[(2S,3S)-3,5-dihydroxy-2-methyl-tetrahydrofuran-3-yl]methyl]-1,4-dihydroxy-anthracen-9,10-dion als roter Feststoff erhalten.
¹H NMR (500 MHz; CDCl₃): δ(ppm) = 13.66 (s, 1H, OH-1); 12.87 (s, 1H, OH-4); 8.36 (m, 2H, H-5 and H-8); 7.85 (m, 2H, H-6 and H-7); 7.29 (s, 1H, H-3); 5.38 (m, 1H, H-4'); 4.01 (q, 1H, J = 6.3 Hz, H-6'); 3.52 (d, 1H, J= 7.3 Hz, OH-4'); 3.39 (s, 1H, OH-2'); 3.07 (d, 1H, J = 13.6 Hz, H-1'/1); 2.90 (d, 1H, J = 13.6 Hz, H-1'/2);; 2.21 (dd, 1H, J = 5.1, 13.3 Hz, H-3'/1); 1.99 (d, 1H, J = 13.3 Hz, H-3'/2); 1.34 (d, 3H, J = 6.3 Hz, CH3).

### Beispiel 19:

### a. Intramolekularer Ringschluss unter Marschalk

### (7R,9S)-6,7,9,11-tetrahydroxy-9-[(1S)-1-hydroxyethyl]-8,10-dihydro-7H-tetracen-5,12-dion; Formel (XVI) ; R₁,R₁₃= H; R₁₂=OH und (7S,9S)-6,7,9,11-tetrahydroxy-9-[(1S)-1-hydroxyethyl]-8,10-dihydro-7H-tetracen-5,12-dion; Formel (XVI) ; R₁,R₁₂= H; R₁₃=OH

Zu einer Lösung von 70 mg *2-[[(2S,3S)-3,5-dihydroxy-2-methyl-tetrahydrofuran-3-yl]methyl]-1,4-dihydroxy-anthracen-9,10-dion* in 5 mL THF und 5 mL MeOH wird tropfenweise unter Argonatmosphäre bei -10 °C eine Lösung von 38 mg NaOH und 49 mg Na₂S₂O₄ in 1,2 mL Wasser zugesetzt. Nach zweistündigem Rühren wird das Reaktionsgemisch durch Einblasen von Luft für 30 min gequencht. Das Reaktionsgemisch wird mit 0.05 N HCl und EtOAc versetzt. Die Phasen werden getrennt, die wässrige Phase wird mit EtOAc extrahiert. Die kombinierten organischen Phasen werden getrocknet und eingedampft. Die erhaltene Mischung wird chromatographisch getrennt (Laufmittel Toluol/Isopropanol 30:1). Es werden 37 mg einer Mischung von *(7R,9S)-6,7,9,11-Tetrahydroxy-9-[(1S)-1-hydroxyethyl]-8,10-dihydro-7H-tetracene-5,12-dion* und *(7S,9S)-6,7,9,11-Tetrahydroxy-9-[(1S)-1-hydroxyethyl]-8,10-dihydro-7H-tetracene-5,12-dion* als roter Feststoff erhalten.

### (7R,9S)-6,7,9,11-tetrahydroxy-9-[(1S)-1-hydroxyethyl]-8,10-dihydro-7H-tetracen-5,12-dion

¹H NMR (500 MHz, CDCl₃) : *δ* = 13.60 (s, 1H, OH-6); 13.33 (s, 1H, OH-11); 8.23 (m, 2H, H-1 and H-4); 7.95 (m, 2H, H-2 and H-3); 5.17 (d, 1H, *J* = 5.7 Hz, OH-7); 5.05 (m, 1H, H-7); 4.85 (d, 1H, *J* = 5.7 Hz, OH-13); 4.45 (s, 1H, OH-9); 3.54 (q, 1H, J = 6.3 Hz, H-13); 2.85 (d, 1H, J = 18.3 Hz, H-10/1); 2.68 (d, 1H, J = 18.3 Hz, H-10/2); 2.14 (m, 1H, H-8/1); 1.75 (m, 1H, H-8/2); 1.14 (d, 3H, J = 6.3 Hz, CH₃).

### (7S,9S)-6,7,9,11-Tetrahydroxy-9-[(1S)-1-hydroxyethyl]-8,10-dihydro-7H-tetracen-5,12-dion

¹H NMR (500 MHz, CDCl₃) : δ = 13.42 (s, 1H, OH-6); 13.30 (s, 1H, OH-11); 8.23 (m, 2H, H-1 and H-4); 7.95 (m, 2H, H-2 and H-3); 5.30 (d, 1H, J = 7.9 Hz, OH-7); 5.14 (s, 1H, OH-9); 5.00 (m, 1H, H-7); 4.81 (d, 1H, J = 5.7 Hz, OH-13); 3.54 (q, 1H, 6.3 Hz, H-13); 2.88 (d, 1H, J = 18.3 Hz, H-10/1); 2.75 (d, 1H, J = 18.3 Hz, H-10/2); 2.14 (m, 1H, H-8/1); 1.75 (m, 1H, H-8/2); 1.16 (d, 3H, J = 6.3 Hz, CH₃).

### b. Intramolekularer Ringschluss unter Marschalk

### (9R)-6,9,11-trihydroxy-9-[(1S)-1-hydroxyethyl]-8,10-dihydro-7H-tetracen-5,12-dion; Formel (XVI); R₁, R₁₂,R₁₃= H

Zu einer Lösung von 0,79 g *2-[[(2S,3S)-3,5-Dihydroxy-2-methyl-tetrahydrofuran-3-yl]methyl]-1,4-dihydroxy-anthracen-9,10-dion* in 31 mL THF und 31 mL MeOH wird bei RT unter Argonatmosphäre eine Lösung von 0,43 g NaOH und 0,56 g Na₂S₂O₄ in 5,3 mL Wasser tropfenweise zugesetzt. Nach 1.5-stündigem Rühren wird das Reaktionsgemisch durch Einblasen von Luft für 30 min gequencht. Das Reaktionsgemisch wird mit 0.05 N HCl und EtOAc versetzt. Die Phasen werden getrennt, die wässrige Phase wird mit EtOAc extrahiert. Die kombinierten organischen Phasen werden getrocknet und eingedampft. Die erhaltene Mischung wird durch Digerieren mit Toluol/EtOAc (1:1) aufgereinigt. Es werden 0.51 g *(9R)-6,9,11-trihydroxy-9-[(1S)-1-hydroxyethyl]-8,10-dihydro-7H-tetracene-5,12-dion* als roter Feststoff erhalten.
¹H NMR (500 MHz; CDCl₃): δ(ppm) = 13.36 (s, 1H, OH-11); 13.35 (s, 1H, OH-6); 8.21 (m, 2H, H-1 and H-4); 7.94 (m, 2H, H-2 and H-3); 4.69 (d, 1H, J = 6.3 Hz, OH-13); 4.28 (s, 1H, OH-9); 3.56 (m, 1H, H-13); 2.82 (d, 1H, J = 18.3 Hz, H-7/1); 2.66 (m, 3H, H-7/2 and H-10); 1.88 (m, 1H, H-8/1); 1.51 (m, 1H, H-8/2);1.14 (d, 3H, J = 6.3 Hz, CH₃).

### Beispiel 20:

### a: Oxidation der Seitenketten-Hydroxylgruppe

### (9R)-9-Acetyl-6,9,11-trihydroxy-8,10-dihydro-7H-tetracene-5,12-dion; Formel (XVII); R₁= H

Zu einer Lösung von 0,8 g (9R)-6,9,11-Trihydroxy-9-[(1S)-1-hydroxyethyl]-8,10-dihydro-7H-tetracen-5,12-dion (0.80 g, 2.258 mmol) in 45 mL DCM werden bei RT unter Argonatmosphäre 1,58 g Dess-Martin Periodinan (97%) zugesetzt. Nach 5 stündigem Rühren wird das Reaktiongemisch mit gesättigter NaHCO₃-Lösung und EtOAc versetzt. Die Phasen werden getrennt, die wässrige Phase wird mit EtOAc extrahiert. Die kombinierten organischen Phasen werden getrocknet und eingedampft. Die erhaltene Mischung wird chromatographisch getrennt (Laufmittel DCM/EtOAc 7:1). Es werden 0.60 g (9R)-9-Acetyl-6,9,11-trihydroxy-8,10-dihydro-7H-tetracene-5,12-dion (1.694 mmol, 75%) als roter Feststoff erhalten.
¹H NMR (500 MHz; CDCl₃) : δ (ppm) = 13.48 (s, 1H, OH-11); 13.47 (s, 1H, OH-6); 8.35 (m, 2H, H-1 and H-4); 7.83 (m, 2H, H-2 and H-3); 3.16 (m, 1H, H-7/1); 3.07 (d, 1H, J = 18 Hz, H-10/1); 2.95 (m, 2H, H-10/2 and H-7/2); 2.39 (s, 3H, CH3); 2.00 (m, 2H, H-8).

### b: Hydroxylierung an C-7

### (7S,9S)-9-acetyl-6,7,9,11-tetrahydroxy-8,10-dihydro-7H-tetracen-5,12-dion; Formel (IV); R₁= R₂= H

Zu einer Suspension von 130 mg *(9R)-9-acetyl-6,9,11-trihydroxy-8,10-dihydro-7H-tetracen-5,12-dion* in 35 mL CCl₄ werden nacheinander 1 mL Wasser, 74 mg NBS und 18 mg AIBN zugegeben. Die Reaktionsmischung wird anschließend 90 Minuten unter Rückfluss erhitzt. Weitere 33 mg NBS werden zugegeben und die Reaktionsmischung weitere 2 Stunden unter Rückfluss erhitzt. Das Reaktionsgemisch wird auf 20 °C abgekühlt und mit 15 mL 10% K₂CO₃-Lösung und 20 mL THF verdünnt. Nach 10 minütigem Rühren wird die wässrige Phase mit 1 N HCl auf pH = 1 gebracht und mit DCM extrahiert. Die vereinigten organischen Phasen werden getrocknet, filtriert und im Vakuum eingedampft. Das Rohprodukt wird chromatographisch auf Silicagel mit Toluol/EtOAc (10/1) gereinigt, wobei 35 mg *(9R)-9-acetyl-6,9,11-trihydroxy-8,10-dihydro-7H-tetracen-5,12-dion,* 16 mg *(7R,9S)-9-acetyl-6,7,9,11-tetrahydroxy-8,10-dihydro-7H-tetracen-5,12-dion* and 48 mg *(7S,9S)-9-acetyl-6,7,9,11-tetrahydroxy-8,10-dihydro-7H-tetracen-5,12-dion* als roter Feststoff erhalten werden.

### (7S,9S)-9-acetyl-6,7,9,11-tetrahydroxy-8,10-dihydro-7H-tetracen-5,12-dion

¹H NMR (500 MHz; CDCl₃): δ(ppm) = 13.53 (s, 1H, OH-6); 13.25 (s, 1H, OH-11); 8.31 (m, 2H, H-1 and H-4); 7.84 (m, 2H, H-2 and H-3); 5.29 (brs, 1H, H-7); 4.58 (s, 1H, OH-9); 3.86 (d, 1H, J = 5.0 Hz, OH-7); 3.17 (dd, 1H, J = 2.2 Hz, 18.6 Hz, H-10/1); 2.94 (d, 1H, J = 18.6 Hz, H-10/2); 2.44 (s, 3H, CH₃); 2.35 (m, 1H, H-8/1); 2.17 (dd, 1H, J = 5.1 Hz, 14.5 Hz, H-8/2).

### (7R,9S)-9-acetyl-6,7,9,11-tetrahydroxy-8,10-dihydro-7H-tetracen-5,12-dion

¹H NMR (500 MHz; CDCl₃): δ(ppm) = 13.93 (s, 1H, OH-6); 13.30 (s, 1H, OH-11); 8.35 (m, 2H, H-1 and H-4); 7.85 (m, 1H, H-2 and H-3); 5.40 (dd, 1H, J = 7.9 Hz, 8.6 Hz, H-7); 4.28 (d, 1H, J = 1.6 Hz, OH-7); 3.90 (s, 1H, OH-9); 3.10 (d, 1H, J = 18.0 Hz, H-10/1); 2.94 (d, 1H, J = 18.0 Hz, H-10/2); 2.41 (s, 3H, CH₃); 2.35 (m , 1H, H-8/1); 2.18 (dd, 1H, J = 9.8 Hz, 13.0 Hz, H-8/2).

### Beispiel 21:

### Epimerisierung von (7R,9S)-9-Acetyl-6,7,9,11-tetrahydroxy-8,10-dihydro-7H-tetracen-5,12-dion

26 mg *(7R,9S)-9-Acetyl-6,7,9,11-tetrahydroxy-8,10-dihydro-7H-tetracen-5,12-dion,* welches mit *(7S,9S)-9-Acetyl-6,7,9,11-tetrahydroxy-8,10-dihydro-7H-tetracene-5,12-dion* verunreinigt sein kann, werden in 1,3 mL TFA aufgelöst. Nach zweistündigem Rühren bei RT wird das Reaktionsgemisch mit Wasser versetzt und mit DCM extrahiert. Die kombinierten organischen Phasen werden getrocknet und eingedampft. Das Rohprodukt wird in 1 mL Aceton aufgelöst. Danach wird gesättigte NaHCO₃-Lösung zugesetzt. Nach 10 min Rühren wird mit DCM extrahiert. Die kombinierten organischen Phasen werden getrocknet und eingedampft. Das erhaltene Produkt wird chromatographisch gereinigt (Laufmittel Toluol/EtOAc 10:1). Es werden 15 mg (*7S,9S*)*-9-Acetyl-6,7,9,11-tetrahydroxy-8,10-dihydro-7H-tetracene-5,12-dion* als roter Feststoff erhalten.
¹H NMR (500 MHz; CDCl₃): δ(ppm) = 13.53 (s, 1H, OH-6); 13.25 (s, 1H, OH-11); 8.31 (m, 2H, H-1 and H-4); 7.84 (m, 2H, H-2 and H-3); 5.29 (brs, 1H, H-7); 4.58 (s, 1H, OH-9); 3.86 (d, 1H, J = 5.0 Hz, OH-7); 3.17 (dd, 1H, J = 2.2 Hz, 18.6 Hz, H-10/1); 2.94 (d, 1H, J = 18.6 Hz, H-10/2); 2.44 (s, 3H, CH₃); 2.35 (m, 1H, H-8/1); 2.17 (dd, 1H, J = 5.1 Hz, 14.5 Hz, H-8/2).

Des Weiteren betrifft die Erfindung folgende Gegenstände/ Verbindungen und Verwendungen:
1'. Verbindungen der allgemeinen Formel (I), 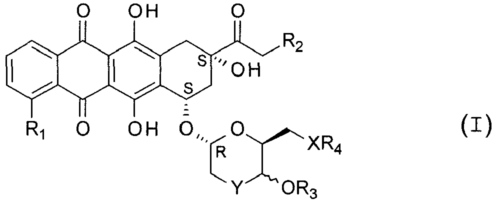 in welcher R₁ ein Wasserstoffatom, eine Hydroxy- oder Methoxy-Gruppe; R₂ ein Wasserstoffatom oder ein Hydroxygruppe ist; R₃ Wasserstoff oder eine geeignete Hydroxyschutzgruppe ist und die gewellte Linie jeweils beide möglichen Konfigurationen von -OR₃ in Bezug zum Grundkörper bedeutet; Y= [C(=0)], [C(=N)-OH] oder [CH-OH], [CH-NR₅R₆] in beiden möglichen stereoisomeren Anordnungen bedeutet, wobei R₅ bzw. R₆ gleich oder verschieden sind und für ein Wasserstoffatom, oder eine geeignete Aminoschutzgruppe wie sie im Stand der Technik etwa aus "Protective Groups in Organic Synthesis" (Greene, Wuts) 4. Auflage, John Wiley&Sons, Inc., Seiten 696 bis 927, bekannt sind, insbesondere eine Trifluoracetyl stehen, unverzweigtes oder verzweigtes Niederalkyl, wobei "Niederalkyl" eine Kohlenstoffanzahl zwischen 1 und 4 bedeutet, oder eine Alkylenkette (-CH₂-CZ₂-CZ₂-CH₂-, -CH₂-CZ₂-CZ₂-CZ₂-CH₂-, -CH₂-O-CZ₂-CH₂-, -CH₂-O-CZ₂-CZ₂-CH₂-, -CH₂-CZ₂-O-CZ₂-CH₂-) wobei Z die Bedeutung von Wasserstoff, Niederalkyl oder Niederalkoxy in beliebiger Kombination bedeutet; worin X= O, NR oder S bedeutet; wobei R Wasserstoff oder Niederalkyl bedeutet; R₄ eine unverzweigte oder verzweigte Alkyl- oder Heteroalkylkette mit einer Kettenlänge von 1 bis 19 Elementen bedeutet, wobei die maximal 6 Heteroatome (O, N, S) beliebig kombinierbar durch mindestens zwei Kohlenstoffatome voneinander getrennt sind.
2'. Verbindungen der allgemeinen Formel (I), in welcher X = O bedeutet und R₁, R₂, R₃, R₄ und Y die in Gegenstand 1' gegebene Bedeutung haben.
3'. Verbindungen der allgemeinen Formel (I) in welcher X = NR bedeutet und R₁, R₂, R₃, R₄ und Y die in Gegenstand 1' gegebene Bedeutung haben.
4'. Verbindungen der allgemeinen Formel (I) in welcher X = S bedeutet und R₁, R₂, R₃, R₄ und Y die in Gegenstand 1' gegebene Bedeutung haben.
5'. Verbindungen gemäß den Gegenständen 1' bis 4' in welcher R₄ mindestens eine Ethylenglykoleinheit (-O-CH₂-CH₂-O-) enthält.
6'. Verfahren zur Herstellung von Verbindungen der allgemeinen Formel (I) gemäß den Gegenständen 1' bis 5', dadurch gekennzeichnet, dass eine offenkettige Zuckerverbindung der allgemeinen Formel (II), 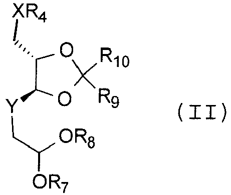 in welcher R₇ und R₈ gleich sind und für Alkyl oder Alkylen mit 2 bis 3 C-Atomen stehen; R₉ und R₁₀ für eine Alkylgruppe mit 1 bis 3 C-Atomen stehen; X und Y die in Formel (I) des Gegenstandes 1' gegebene Bedeutung haben; R₄ Wasserstoff oder eine geeignete Hydroxyschutzgruppe wie sie im Stand der Technik etwa aus "Protective Groups in Organic Synthesis" (Greene, Wuts) 4. Auflage, John Wiley&Sons, Inc., Seiten 16 bis 288, bekannt sind, insbesondere eine Benzoylgruppe bedeutet, oder die für R₄ in Formel (I) gegebene Bedeutung hat, zu einer Zuckerverbindung der allgemeinen Formel (III), 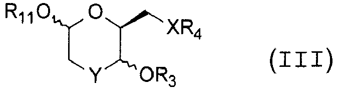 in welcher X, Y und R₄ die in Formel (II) gegebene Bedeutung haben, R₃ und R₁₁ eine für die Glycosylierung bekannte Aktivierungsgruppe [C(=O)PHNO₂, OTFA] darstellen und die gewellten Linien jeweils beide möglichen Konfigurationen von -OR₃ und -OR₁₁ in Bezug auf den Grundkörper bedeuten, zyklisiert wird und mit einem vom Anthrachinon abgeleiteten Aglycon der allgemeinen Formel (IV), 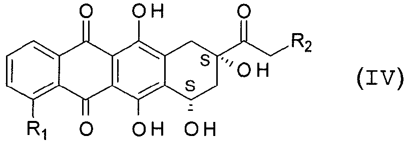 in welcher R₁ und R₂ die in Gegenstand 1' angegebene Bedeutung haben, zur Reaktion gebracht wird und anschließend die am Zucker noch vorhandenen Schutzgruppen unter basischen Bedingungen, bevorzugt mit Natronlauge abgespalten werden, um Verbindungen der Allgemeinen Formel (I) des Gegenstandes 1' zu erhalten.
7'. Verfahren gemäß Gegenstand 6', dadurch gekennzeichnet, dass die zu zyklisiernde Verbindung der allgemeinen Formel (II) dadurch hergestellt wird, dass ein C2-Baustein der allgemeinen Formel (V), 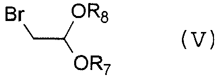 in welcher R₇ und R₈ die in Formel (II) gegebene Bedeutung haben, mit einem geschützten Derivat der L-Threose (enatiomerenreiner C4-Baustein) der allgemeinen Formel (VI), 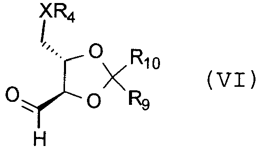 in welcher X, R₄, R₉ und R₁₀ die in Formel (II) gegebene Bedeutung haben, nach bekannten Methoden verknüpft wird,
   wobei die C-C Knüpfung mittels Grignard-Reaktion in einem aprotischen Lösungsmittel wie Tetrahydrofuran bevorzugt wird und das daraus resultierende Additionspordukt der allgemeinen Formel (II)in welcher [Y= CH-OH] bedeutet, kann anschließend durch bekannte Verfahren wie Oxidation mit Chromverbindungen, vorzugsweise jedoch mittels Swern-Oxidation zum Keton [Y= C(=O)] der allgemeinen Formel (II), oxidiert werden,
   um anschließend nach bekannten Methoden z.B. durch reduktive Aminierung den Stickstoff an Position 3 einzuführen, vorzugsweise jedoch durch Herstellung eines Oxims [Y= C(=N)-OH] der allgemeinen Formel (II), welches in Folge reduziert wird, jedoch zuvor für den Fall dass R₄ Wasserstoff oder eine HydroxySchutzgruppe bedeutet, wenn nötig entschützt und gemäß der Beschreibung für R₄ in Formel (I), derivatisiert werden kann, wobei die Einführung einer unverzweigten oder verzweigten Alkyl-oder Heteroalkylkette an X nach bekannten Methoden z.B. Finkelstein-Reaktion, vorzugsweise jedoch über eine nukleophile Substitution, wo die einzuführende Kette zuvor mit einer guten Abgangsgruppen wie Tosylat oder Mesylat aktiviert wird, erfolgt,
   und anschließend Oxime der allgemeinen Formel (II)[Y= C(=N)-OH] reduziert werden, wobei bekannte Methoden zum Einsatz kommen können, bevorzugt ein komplexes Hydrid in einem aprotischen Lösungsmittel wie Toluol oder THF, wobei Amine der allgemeinen Formel (II) [Y= CH-NH₂] entstehen, die an dieser Stelle gegebenenfalls weiter derivatisiert oder geschützt [Y= CH-NR₅R₆] werden, wobei R₅ und R₆ die in der allgemeinen Formel (I) angegebene Bedeutung haben.
8'. Verbindungen der allgemeinen Formel (III) 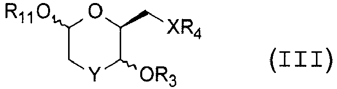 worin X, Y und R₄ die in Formel (II) gegebene Bedeutung haben und R₆ und R₁₁ Wasserstoff oder eine für die Glycosylierung bekannte Aktivierungsgruppe [OC(=O)PHNO₂, OTFA] darstellt und die gewellten Linien jeweils beide möglichen Konfigurationen von -OR₃ und -OR₁₁ in Bezug auf den Grundkörper bedeuten.
9'. Verbindungen nach Gegenstand 8', dadurch gekennzeichnet, dass R₃ und R₁₁ jeweils Wasserstoff bedeutet, Y= [CH-NR₅R₆] bedeutet, wobei R₅ Wasserstoff und R₆ TFA bedeutet, X= O bedeutet und R₄ mindestens eine Ethylenglykoleinheit (-O-CH₂-CH₂-O-) enthält.
10'. Verbindungen nach Gegenstand 9', dadurch gekennzeichnet, dass R₃ und R₁₁ jeweils p-Nitrobenzoyl bedeutet.
11'. Verbindungen der allgemeinen Formel (II), 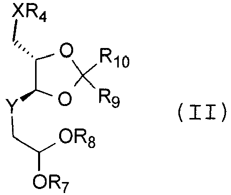 in welcher R₇ und R₈ gleich sind und für Alkyl oder Alkylen mit 2 bis 3 C-Atomen stehen; R₉ und R₁₀ eine Alkylgruppe mit 1 bis 3 C-Atomen; X und Y die in Formel (I) gegebene Bedeutung haben; R₄ Wasserstoff oder eine geeignete Hydroxyschutzgruppe wie sie im Stand der Technik etwa aus "Protective Groups in Organic Synthesis" (Greene, Wuts) 4. Auflage, John Wiley&Sons, Inc., Seiten 16 bis 288, bekannt sind, insbesondere eine Benzoylgruppe bedeutet, oder die die in Formel (I) gegebene Bedeutung hat.
12'. Verwendung von Verbindungen der allgemeinen Formel I alleine oder in Kombination mit andere Wirkstoffen in Arzneimitteln.
13'. Die Verwendung von Verbindungen der allgemeinen Formel III als Strukturelement in Arzneistoffen.

## Patentansprüche

1. Verbindungen der allgemeinen Formel (IIIa/b) 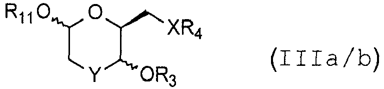 worin R₃ Wasserstoff, Trifluoracetyl (C(=O)CF₃) oder *p*-Nitrobenzoyl (C(=O)PhNO₂) ist; Y = [C(=O)], [C(=N)-OH] oder [CH-OH], [CH-NR₅R₆] in beiden möglichen stereoisomeren Anordnungen bedeutet, wobei R₅ und R₆ entweder jeweils für ein Wasserstoffatom stehen oder für ein Wasserstoffatom und eine Trifluoracetylgruppe (TFA) stehen; worin X = O, S oder NR, mit R = Wasserstoff oder einer C₁ bis C₄ Alkylgruppe bedeutet; R₄ eine unverzweigte oder verzweigte Alkyl- oder Heteroalkylkette mit einer Kettenlänge von 1 bis 19 Elementen bedeutet, wobei maximal 6 Heteroatome (O, N, S) beliebig kombinierbar durch mindestens zwei Kohlenstoffatome voneinander getrennt sind, R₁₁ Wasserstoff, Trifluoracetyl (C(=O)CF₃) oder p-Nitrobenzoyl [C(=O)PhNO₂] oder ein tetracyclisches Aglycon vom Strukturtyp der Anthracycline bedeutet und die gewellten Linien jeweils beide möglichen Konfigurationen von -OR₃ und -OR₁₁ in Bezug auf den Grundkörper bedeuten.

2. Verbindungen nach Anspruch 1, der allgemeinen Formel (IIIc) 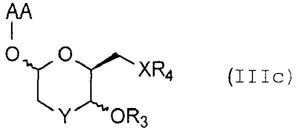 **dadurch gekennzeichnet, dass** AA ein tetracyclisches Aglycon vom Strukturtyp der Anthracycline bedeutet.

3. Verbindungen nach Anspruch 1, der allgemeinen Formel (I), 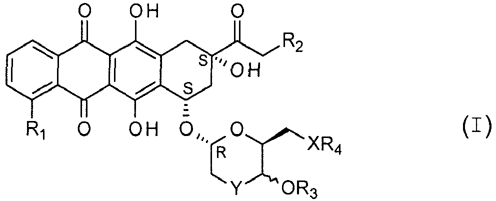 in welcher R₁ ein Wasserstoffatom, eine Hydroxy- oder Methoxy-Gruppe, ein Halogenatom oder eine NO₂-Gruppe ist; R₂ ein Wasserstoffatom, eine Hydroxy- oder Methoxygruppe, ein Acyl- oder Aroylrest ist; R₃ Wasserstoff, Trifluoracetyl (C(=O)CF₃) oder *p*-Nitrobenzoyl (C(=O)PhNO₂) ist und die gewellte Linie jeweils beide möglichen Konfigurationen von -OR₃ in Bezug zum Grundkörper bedeutet; Y= [C(=O)], [C(=N)-OH] oder [CH-OH], [CH-NR₅R₆] in beiden möglichen stereoisomeren Anordnungen bedeutet, wobei R₅ und R₆ entweder jeweils für ein Wasserstoffatom stehen oder für ein Wasserstoffatom und eine Trifluoracetylgruppe (TFA) stehen; worin X = O, S oder NR, mit R = Wasserstoff oder einer C₁ bis C₄ Alkylgruppe bedeutet; R₄ eine unverzweigte oder verzweigte Alkyl- oder Heteroalkylkette mit einer Kettenlänge von 1 bis 19 Elementen bedeutet, wobei maximal 6 Heteroatome (O, N, S) beliebig kombinierbar durch mindestens zwei Kohlenstoffatome voneinander getrennt sind.

4. Verbindungen gemäß Anspruch 3, wobei diese die folgende Formel (Ia) aufweisen 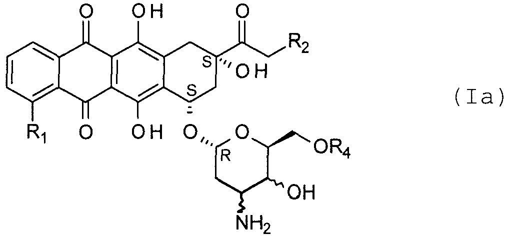 in welcher R₁ ein Wasserstoffatom, eine Hydroxy- oder Methoxy-Gruppe; R₂ ein Wasserstoffatom oder ein Hydroxygruppe ist; die Aminogruppe als auch die Hydroxygruppe in beiden möglichen stereochemischen Anordungen vorliegen können; R₄ die Gruppe (CH₂-CH₂-O)ₙ- mit n = 1 bis 6 bedeutet, wobei am terminalen Sauerstoffatom der (CH₂-CH₂-O)ₙ-Gruppe ein Wasserstoffatom oder eine C₁ bis C₄ Alkylgruppe gebunden ist.

5. Verbindungen nach Anspruch 3, in welcher X = O bedeutet und R₁, R₂, R₃, R₄ und Y die in Anspruch 3 gegebene Bedeutung haben oder in welcher X= NR bedeutet und R, R₁, R₂, R₃, R₄ und Y die in Anspruch 3 gegebene Bedeutung haben oder in welcher X= S bedeutet und R₁, R₂, R₃, R₄ und Y die in Anspruch 3 gegebene Bedeutung haben.

6. Verbindungen nach Anspruch 1, **dadurch gekennzeichnet, dass** R₃ und R₁₁ jeweils Wasserstoff bedeutet, Y= [CH-NR₅R₆] bedeutet, wobei R₅ Wasserstoff und R₆ TFA bedeutet, X= O bedeutet und R₄ die Gruppe (CH₂-CH₂-O)ₙ- mit n = 1 bis 6 bedeutet, wobei am terminalen Sauerstoffatom der (CH₂-CH₂-O)ₙ-Gruppe ein Wasserstoffatom oder eine C₁ bis C₄ Alkylgruppe gebunden ist.

7. Verbindungen nach Anspruch 6, **dadurch gekennzeichnet, dass** R₃ und R₁₁ jeweils p-Nitrobenzoyl bedeutet.

8. Verbindungen der allgemeinen Formel (II), 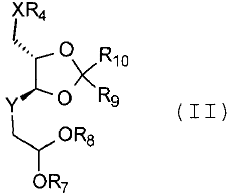
in welcher R₇ und R₈ gleich sind und für Alkyl oder Alkylen mit 2 bis 3 C-Atomen stehen;
R₉ und R₁₀ eine Alkylgruppe mit 1 bis 3 C-Atomen; X, Y und R₄ die in Anspruch 3 gegebene Bedeutung haben.

9. Verfahren zur Herstellung von Verbindungen der allgemeinen Formel (IIIc) gemäß Anspruch 2 vorzugsweise von Verbindungen der Formel (I) gemäß Anspruch 3 insbesondere Verbindungen der Formel (Ia) gemäß Anspruch 4, **dadurch gekennzeichnet, dass** eine offenkettige Zuckerverbindung der allgemeinen Formel (II), 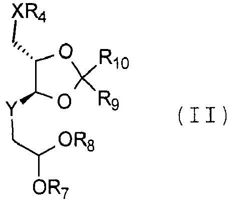 in welcher R₇ und R₈ gleich sind und für Alkyl oder Alkylen mit 2 bis 3 C-Atomen stehen; R₉ und R₁₀ für eine Alkylgruppe mit 1 bis 3 C-Atomen stehen; X, Y und R₄ die in Anspruch 3 gegebene Bedeutung haben, durch Spaltung der Diol und Aldehyd-Schutzgruppe, zu einer Pyranose der allgemeinen Formel (III), 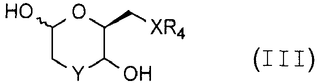 in welcher X, Y und R₄ die in Anspruch 3 gegebene Bedeutung haben, umgesetzt wird, die in weiterer Folge, geschützt wird, vorzugsweise als O-Trifluoracetyl oder O-para-Nitrobenzoyl, wobei Verbindungen der allgemeinen Formel (IIIa) bzw. (IIIb) entstehen, 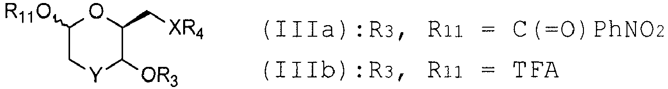 in welchen X, Y und R₄ die in Anspruch 3 gegebene Bedeutung haben und die im Anschluss mit einem tetracyklischen Aglycon vom Strukturtyp der Anthracycline (AA), zu einer Verbindung der allgemeinen Formel IIIc, 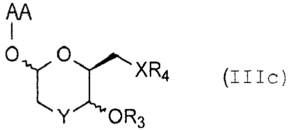 in welcher AA ein tetracyclisches Aglycon vom Strukturtyp der Anthrycycline ist und X, Y, R₃ und R₄ die in Formel I gemäß Anspruch 3 angegebene Bedeutung haben, glycosyliert wird, jedoch in der bevorzugten Ausführungsform werden die aktivierten Pyranosen der allgemeinen Formel (IIIa) oder (IIIb) mit einem vom Anthrachinon abgeleiteten Aglycon der allgemeinen Formel (IV), 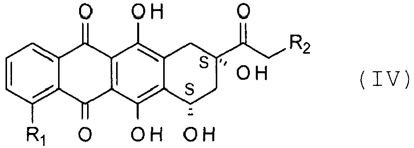 in welcher R₁ und R₂ die in Formel (I) gemäß Anspruch 3 angegebene Bedeutung haben, zur Glykosidierungsreaktion gebracht, wobei eine Reaktionsaktivierung erfolgt, wobei insbesondere Verbindungen der Allgemeinen Formel (Ia) erhalten werden, 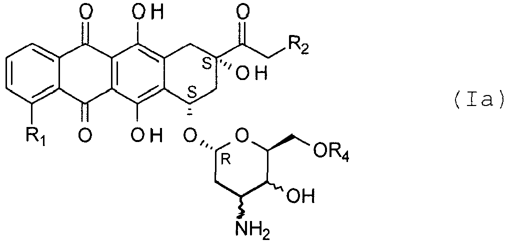 in welcher R₁ ein Wasserstoffatom, eine Hydroxy- oder Methoxy-Gruppe, ein Halogenatom oder eine NO₂-Gruppe ist; R₂ ein Wasserstoffatom, eine Hydroxy- oder Methoxygruppe, ein Acyl- oder Aroylrest ist, R₄ die Bedeutung -(CH₂-CH₂O)ₙ- mit n= 1 bis 6 bedeutet und worin am terminalen Sauerstoffatom der Kette ein Wasserstoffatom oder eine C₁ bis C₄ Alkylgruppe, angebracht ist.

10. Verfahren gemäß Anspruch 9, **dadurch gekennzeichnet, dass** Verbindungen der allgemeinen Formel (II) dadurch hergestellt werden, dass ein C2-Baustein der allgemeinen Formel (V), 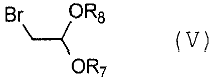 in welcher R₇ und R₈ die in Formel (II) gegebene Bedeutung haben, mit einem geschützten Derivat der L-Threose (enatiomerenreiner C4-Baustein) der allgemeinen Formel (VI), 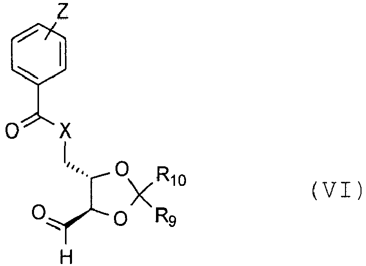 in welcher X, R₉, R₁₀ die in Formel (II) gegebene Bedeutung haben und Z = Wasserstoff, oder mindestens reine oder mehrere, vorzugsweise eine oder zwei, Methyl-, Fluor-, Chlor-, Brom- oder Nitrogruppen bedeutet, gekoppelt wird, und das daraus resultierende Additionsprodukt der allgemeinen Formel (VII) 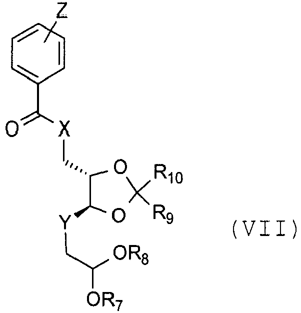 in welcher R₇, R₈, R₉, R₁₀ die in Formel (II) gegebene Bedeutung haben, und Z = Wasserstoff, oder mindestens oder mehrere, vorzugsweise eine oder zwei, Methyl-, Fluor-, Chlor-, Brom- oder Nitrogruppen bedeutet und Y= CH-OH bedeutet, durch Oxidation zum Keton [Y= C(=O)] der allgemeinen Formel (VII) oxidiert wird und anschließend die Einführung des Stickstoffs, bevorzugt über ein Oxim [Y= C(=N)-OH] der allgemeinen Formel (VII) erfolgt, an welchem in weiterer Folge die Schutzgruppe an X abgespalten wird und R₄ gemäß der in Formel (I) gegebenen Bedeutung eingeführt wird, wobei die Einführung einer unverzweigten oder verzweigten Alkyl- oder Heteroalkylkette an X durch Substitution, vorzugsweise mithilfe einer nicht nukleophilen Base erfolgt, wobei die einzuführende Alkyl- oder Heteroalkylkette zuvor mit einer Abgangsgruppe aktiviert wird und das dabei entstehende Oxim [Y= C(=N)-OH] der allgemeinen Formel (II) im Anschluss mit einem Metallhydrid reduziert wird, wobei Amine [Y= CH-NH₂] der allgemeinen Formel (II) entstehen, die anschließend geschützt werden, wobei eine Zuckerverbindung der allgemeinen Formel (II) [Y= CH-NR₅R₆] entsteht, in welcher R₅/R₆ verschieden sind und die in Formel (I) angegebene Bedeutung haben.

11. Pharmazeutische Zusammensetzung umfassend eine oder mehrere Verbindungen der Formel (I), (Ia) und (IIIc) gemäß Ansprüche 2-4 und optional einen oder mehrere pharmazeutisch annehmbare Stoffe, ausgewählt aus Hilfsstoffen, Träger, Verdünnungsmittel und Lösungsmitteln.

12. Pharmazeutischer Kit umfassend (i) eine oder mehrere Verbindungen der Formel (I), (Ia) oder (IIIc) gemäß Ansprüche 2-4, oder eine pharmazeutische Zusammensetzung gemäß Anspruch 11 und (ii) mindestens einen antiproliferativen oder cytotoxischen Wirkstoff.

13. Verbindungen der Formel (I), (Ia) oder (IIIc) gemäß Ansprüche 2-4, pharmazeutische Zusammensetzung gemäß Anspruch 11 oder pharmazeutischer Kit gemäß Anspruch 12 zur Anwendung als Medikament oder als Werkzeug in der biomedizinischen Forschung.

14. Verbindungen der Formel (I), (Ia) oder (IIIc) gemäß Ansprüche 2-4, pharmazeutische Zusammensetzung gemäß Anspruch 11 oder pharmazeutischer Kit gemäß Anspruch 12 zur Anwendung bei der Behandlung von Krankheiten oder Krankheitszuständen, die zumindest teilweise durch Therapie gelindert werden können.

15. Verbindungen der Formel (I), (Ia) oder (IIIc) gemäß Ansprüche 2-4, pharmazeutische Zusammensetzung oder pharmazeutischer Kit zur Anwendung gemäß Anspruch 14, wobei die Krankheiten oder Krankheitszustände ausgewählt sind aus proliferierenden Krankheiten, vorzugsweise Krebs.

## Claims

1. Compounds with the general formula (IIIa/b) 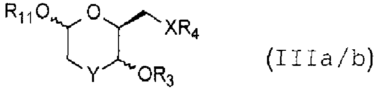 in which R₃ is hydrogen, trifluoroacetyl (C(=O)CF₃) or *p*-nitrobenzoyl (C(=O)PhNO2); Y= [C(=O)], [C(=N)-OH] or [CH-OH], [CH-NR₅R₆] in both possible stereoisomer arrangements, wherein R₅ and R₆ each stand either for a hydrogen atom or for a hydrogen atom and a trifluoroacetyl group (TFA); wherein X = O, S or NR, with R = hydrogen or a C₁ to C₄ alkyl group; R₄ is an unbranched or branched alkyl or heteroalkyl chain with a chain length of 1 to 19 elements, wherein a maximum of 6 heteroatoms (O, N, S) in any combination are separated from each other by at least two carbon atoms, R₁₁ is hydrogen, trifluoroacetyl (C(=O)CF₃) or *p-*nitrobenzoyl [C(=O)PhNO₂] or a tetracyclic aglycone of the structure type of the anthracyclines and the wavy lines are each both possible configurations of -OR₃ and -OR₁₁ in relation to the base body.

2. Compounds according to claim 1, with the general formula (IIIc) 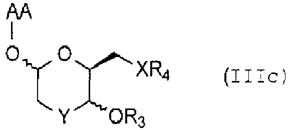 **characterised in that** AA is a tetracyclic aglycone of the structure type of the anthracyclines.

3. Compounds according to claim 1, with the general formula (I), 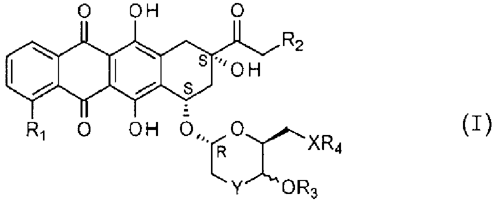 in which R₁ is a hydrogen atom, a hydroxyl or methoxy group, a halogen atom or a NO₂ group; R₂ is a hydrogen atom, a hydroxyl or methoxy group, an acyl or aroyl rest; R₃ is hydrogen, trifluoroacetyl (C(=O)CF₃) or *p*-nitrobenzoyl (C(=O)PhNO₂) and the wavy line is both possible configurations of -OR3 in relation to the base body; Y= [C(=O)], [C(=N)-OH] or [CH-OH], [CH-NR₅R₆] in both possible stereoisomer arrangements, wherein R₅ and R₆ are each either a hydrogen atom or a hydrogen atom and a trifluoroacetyl group (TFA); wherein X = O, S or NR, with R = hydrogen or a C₁ to C₄ alkyl group; R₄ is an unbranched or branched alkyl or heteroalkyl chain with a chain length of 1 to 19 elements, wherein a maximum of 6 heteroatoms (O, N, S) in any combination are separated from each other by at least two carbon atoms.

4. Compounds according to claim 3, wherein these have the following formula (Ia) 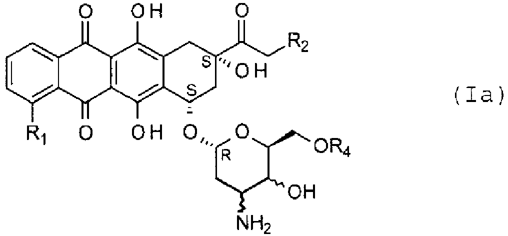 in which R₁ is a hydrogen atom, a hydroxyl or methoxy group; R₂ is a hydrogen atom or a hydroxyl group; the amino group as well as the hydroxyl group can also be present in both stereochemical arrangements; R₄ is the group (CH₂-CH₂-O)ₙ- with n = 1 to 6, wherein a hydrogen atom or a C₁ to C₄ alkyl group is bound to the terminal oxygen atom of the (CH₂-CH₂-O)ₙ group.

5. Compounds according to claim 3, in which X = O and R₁, R₂, R₃, R₄ and Y have the meaning stated in claim 3 or in which X= NR and R, R₁, R₂, R₃, R₄ and Y have the meaning stated in claim 3 or in which X= S and R₁, R₂, R₃, R₄ and Y have the meaning stated in claim 3.

6. Compounds according to claim 1, **characterised in that** R₃ and R₁₁ are each hydrogen, Y= [CH-NR₅R₆], wherein R₅ is hydrogen and R₆ is TFA, X= O and R₄ is the group (CH₂-CH₂-O)ₙ-with n = 1 to 6, wherein a hydrogen atom or a C₁ to C₄ alkyl group is bound to the terminal oxygen atom of the (CH₂-CH₂-O)ₙ group.

7. Compounds according to claim 6, **characterised in that** R₃ and R₁₁ are each *p*-nitrobenzoyl.

8. Compounds with the general formula (II), 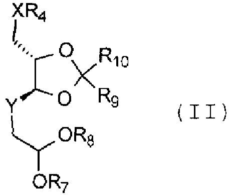
in which R₇ and R₈ are identical and stand for alkyl or alkylene with 2 to 3 C atoms;
R₉ and R₁₀ are an alkyl group with 1 to 3 C atoms; X, Y and R₄ have the meaning stated in claim 3.

9. Methods for producing compounds with the general formula (IIIc) according to claim 2, preferably of compounds with the formula (I) according to claim 3, in particular compounds with the formula (Ia) according to claim 4, **characterised in that** an open-chain sugar compound with the general formula (II), 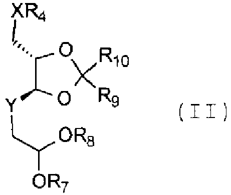 in which R₇ and R₈ are identical and stand for alkyl or alkylene with 2 to 3 C atoms; R₉ and R₁₀ stand for an alkyl group with 1 to 3 C atoms; X, Y and R₄ have the meaning stated in claim 3, are created through splitting the diol and aldehyde protection group into a pyranose with the general formula (III), 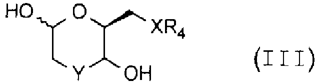 in which X, Y and R₄ have the meaning stated in claim 3, which is converted, which is subsequently protected, preferably as an O-trifluoroacetyl or an O-para-nitrobenzoyl, wherein compounds with the general formula (IIIa) or (IIIb) are created, 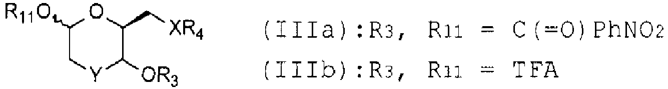 in which X, Y and R₄ have the meaning stated in claim 3 and which are subsequently glycolised with a tetracyclic aglycone of the structure type of the anthracyclines (AA), to a compound with the general formula IIIc, 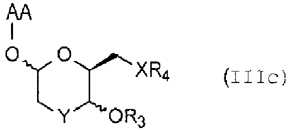 in which AA is a tetracyclic aglycone of the structure type of the anthrycyclines and X, Y, R₃ and R4 have the meaning stated in formula I according to claim 3, although the activated pyranoses with the general formula (IIIa) or (IIIb) with an aglycone derived from anthrachinon with the general formula (IV), 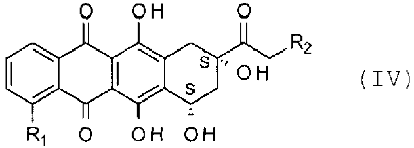 in the preferred embodiment, in which R₁ and R₂ have the meaning stated in formula (I) according to claim 3, are brought to a glycosidation reaction, wherein a reaction activation occurs, wherein in particular compounds with the general formula (Ia) are obtained, 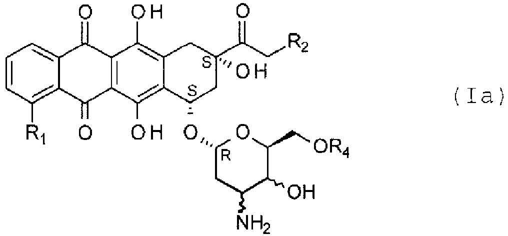 in which R₁ is a hydrogen atom, a hydroxyl or methoxy group, a halogen atom or a NO2 group; R₂ is a hydrogen atom, a hydroxyl or methoxy group, an acyl or aroyl rest, R₄ has the meaning -(CH2-CH₂-O)ₙ- with n= 1 to 6 and wherein a hydrogen atom or a C₁ to C₄ alkyl group is attached to the terminal oxygen atom of the chain.

10. Method according to claim 9, **characterised in that** compounds with the general formula (II) are produced **in that** a C₂ component with the general formula (V), 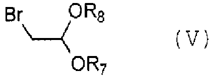 in which R₇ and R₈ have the meaning stated in formula (II), is coupled with a protected derivative of the L-threose (enatiopure C4 component) with the general formula (VI), 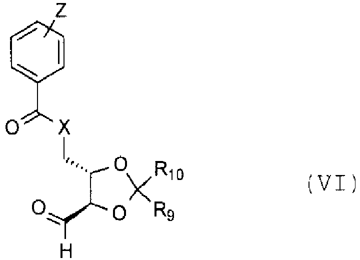 in which X, R₉, R₁₀ have the meaning stated in formula (II) and Z = hydrogen, or mean at least one or more, preferably one or two, methyl, fluor, chlorine, bromine or nitro groups, and the addition product resulting from this with the general formula (VII) 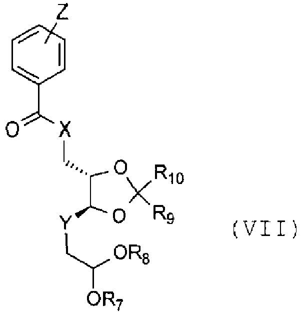 in which R₇, R₈, R₉, R₁₀ have the meaning stated in formula (II), and Z = hydrogen, or mean at least one or more, preferably one or two, methyl, fluor, chlorine, bromine or nitro groups and Y= CH-OH, are oxidised through oxidation to a ketone [Y= C(=O)] with the general formula (VII), and the introduction of nitrogen then takes place, preferably via an oxime [Y= C(=N)-OH] with the general formula (VII), whereafter the protection group is then split off X and R₄ is introduced according to the meaning stated in formula (I), wherein the introduction of an unbranched or branched alkyl or heteroalkyl chain to X takes place through substitution, preferably with the aid a non-nucleophilie base, wherein the alkyl or heteroalkyl chain to be introduced is first activated with a leaving group and the oxime [Y= C(=N)-OH] with the general formula (II) created in this way is subsequently reduced with a metal hydride, wherein amines [Y= CH-NH₂] with the general formula (II) are created, which are then protected, wherein a sugar compound with the general formula (II) [Y= CH-NR₅R₆] is created, in which R₅/R₆ are different and have the meaning stated in formula (I).

11. Pharmaceutical composition comprising one or more compounds with the formula (I), (Ia) and (IIIc) according to claims 2-4 and optionally one or more pharmaceutically acceptable substances, selected from additives, substrates, diluents and solvents.

12. Pharmaceutical kit comprising (i) one or more compounds with the formula (I), (Ia) or (IIIc) according to claims 2-4, or a pharmaceutical composition according to claim 11 and (ii) at least one antiproliferative or cytotoxic active substance.

13. Compounds with the formula (I), (Ia) or (IIIc) according to claims 2-4, pharmaceutical composition according to claim 11 or pharmaceutical kit according to claim 12 for use as a medication or as a tool for biomedical research.

14. Compounds with the formula (I), (Ia) or (IIIc) according to claims 2-4, pharmaceutical composition according to claim 11 or pharmaceutical kit according to claim 12 for use during the treatment of conditions or medical conditions which can at least be alleviated with therapy.

15. Compounds with the formula (I), (Ia) or (IIIc) according to claims 2-4, pharmaceutical composition or pharmaceutical kit for use according to claim 14, wherein the conditions or medical conditions are selected from proliferating conditions, preferably cancer.

## Revendications

1. Composés de formule générale (IIIa/b) 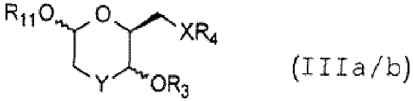 dans laquelle R₃ est un atome d'hydrogène, un groupe trifluoroacétyle (C(=O)CF₃) ou p-nitrobenzoyle (C(C=O)PhNO₂) ; Y = [C(=O=)], [C(=N)-OH] ou représente [CH-OH], [CH-NR₅R₆] en les deux configurations stéréoisomères possibles, R₅ et R₆ soit représentant chacun un atome d'hydrogène soit représentant un atome d'hydrogène et un groupe trifluoroacétyle (TFA) ; dans laquelle X = O, S ou NR, où R représente un atome d'hydrogène ou un groupe alkyle en C₁-C₄ ; R₄ représente une chaîne alkyle ou hétéroalkyle ramifiée ou non ramifiée, ayant une longueur de chaîne de 1 à 19 éléments, au maximum 6 hétéroatomes (O, N, S) étant séparés les uns des autres de façon librement combinable par au moins deux atomes de carbone, R₁₁ représente un atome d'hydrogène, un groupe trifluoroacétyle (C(=O)CF₃) ou *p*-nitrobenzoyle [C(=O)PhNO₂] ou un aglycone tétracyclique du type structural des anthracyclines et les traits ondulés représentent chacun les deux configurations possibles de -OR₃ et -OR₁₁ par rapport au corps de base.

2. Composés selon la revendication 1, de formule générale (IIIc) 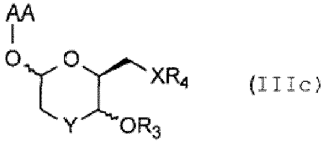 **caractérisés en ce que** AA représente un aglycone tétracyclique du type structural des anthracyclines.

3. Composés selon la revendication 1, de formule générale (I), 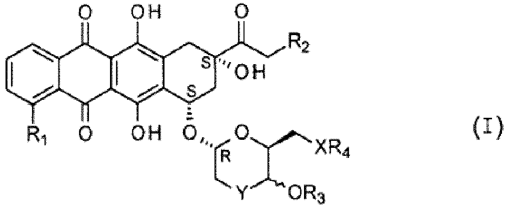 dans laquelle R₁ est un atome d'hydrogène, un groupe hydroxy ou méthoxy, un atome d'halogène ou un groupe NO₂ ; R₂ représente un atome d'hydrogène, un groupe hydroxy ou méthoxy, un radical acyle ou aroyle ; R₃ est un atome d'hydrogène, un groupe trifluoroacétyle (C(=O)CF₃) ou p-nitrobenzoyle (C(C=O)PhNO₂) et le trait ondulé représente chaque fois les deux configurations possible de -OR₃ par rapport au corps de base ; Y = [C (=O=)], [C(=N)-OH] ou représente [CH-OH], [CH-NR₅R₆] en les deux configurations stéréoisomères possibles, R₅ et R₆ soit représentant chacun un atome d'hydrogène soit représentant un atome d'hydrogène et un groupe trifluoroacétyle (TFA) ; dans laquelle X = O, S ou NR, où R représente un atome d'hydrogène ou un groupe alkyle en C₁-C₄ ; R₄ représente une chaîne alkyle ou hétéroalkyle ramifiée ou non ramifiée, ayant une longueur de chaîne de 1 à 19 éléments, au maximum 6 hétéroatomes (O, N, S) étant séparés les uns des autres de façon librement combinable par au moins deux atomes de carbone.

4. Composés selon la revendication 3, ces derniers présentant la formule (Ia) suivante 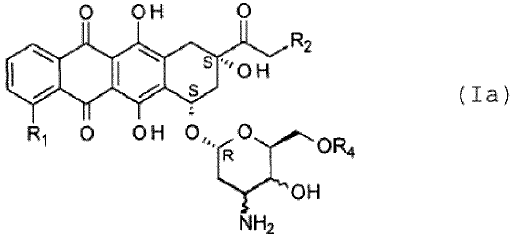 dans laquelle R₁ est un atome d'hydrogène, un groupe hydroxy ou méthoxy ; R₂ est un atome d'hydrogène ou un groupe hydroxy ; le groupe amino ainsi que le groupe hydroxy peuvent se trouver en les deux configurations stéréochimiques possibles ; R₄ représente le groupe (CH₂-CH₂-O)ₙ- où n = 1 à 6, un atome d'hydrogène ou un groupe alkyle en C₁-C₄ étant lié à l'atome d'oxygène terminal du groupe (CH₂-CH₂-O)ₙ-.

5. Composés selon la revendication 3, dans lesquels X représente O et R₁, R₂, R₃, R₄ et Y ont la signification donnée dans la revendication 3 ou dans lesquels X représente NR et R, R₁, R₂, R₃, R₄ et Y ont la signification donnée dans la revendication 3 ou dans lesquels X représente S et R₁, R₂, R₃, R₄ et Y ont la signification donnée dans la revendication 3.

6. Composés selon la revendication 1, **caractérisés en ce que** R₃ et R₁₁ représentent chacun un atome d'hydrogène, Y représente [CH-NR₅R₆], R₅ représentant un atome d'hydrogène et R₆ représentant un groupe TFA, X représente O et R₄ représente le groupe (CH₂-CH₂-O)ₙ- où n = 1 à 6, un atome d'hydrogène ou un groupe alkyle en C₁-C₄ étant lié à l'atome d'oxygène terminal du groupe (CH₂-CH₂-O)ₙ-.

7. Composés selon la revendication 6, **caractérisés en ce que** R₃ et R₁₁ représentent chacun un groupe p-nitrobenzoyle.

8. Composés de formule générale (II), 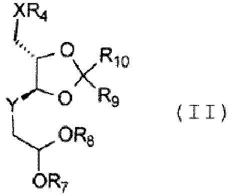
dans laquelle R₇ et R₈ sont identiques et représentent un groupe alkyle ou alkylène ayant 2 ou 3 atomes de carbone ;
R₉ et R₁₀ représentent un groupe alkyle ayant de 1 à 3 atomes de carbone ; X, Y et R₄ ont la signification donnée dans la revendication 3.

9. Procédé pour la préparation de composés de formule générale (IIIc) selon la revendication 2, de préférence de composés de formule (I) selon la revendication 3, en particulier de composés de formule (Ia) selon la revendication 4, **caractérisé en ce qu'**on convertit un composé glucidique à chaîne ouverte de formule générale (II), 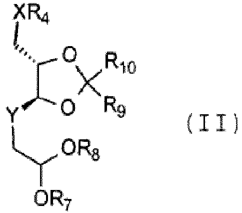 dans laquelle R₇ et R₈ sont identiques et représentent un groupe alkyle ou alkylène ayant 2 ou 3 atomes de carbone ; R₉ et R₁₀ représentent un groupe alkyle ayant de 1 à 3 atomes de carbone ; X, Y et R₄ ont la signification donnée dans la revendication 3, par séparation du diol et du groupe protecteur de fonction aldéhyde, en un pyranose de formule générale (III), 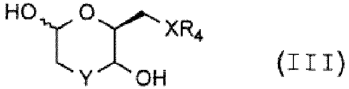 dans laquelle X, Y et R₄ ont la signification donnée dans la revendication 3, qui est ensuite protégé, de préférence sous forme d'O-trifluoroacétyle ou d'O-para-nitrobenzoyle, de sorte qu'il en résulte des composés de formule générale (IIIa) ou (IIIb), 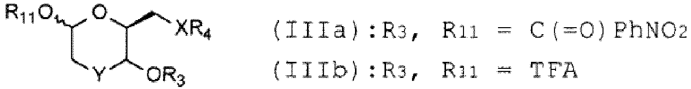 formules dans lesquelles X, Y et R₄ ont la signification donnée dans la revendication 3, et qui est ensuite glycosylé avec un aglycone tétracyclique du type structural des anthracyclines (AA), en un composé de formule générale IIIc, 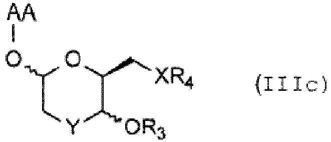 dans laquelle AA est un aglycone tétracyclique du type structural des anthracyclines et X, Y, R₃ et R₄ ont la signification indiquée dans la formule I selon la revendication 3.
mais dans le mode de réalisation préféré on met en la réaction de glycosylation les pyranoses activés de formule générale (IIIa) ou (IIIb) avec un aglycone dérivé de l'anthraquinone, de formule générale (IV), 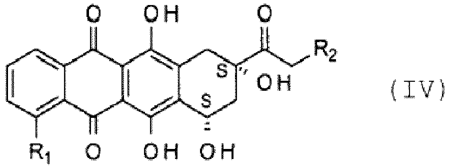 dans laquelle R₁ et R₂ ont la signification indiquée dans la formule (I) selon la revendication 3, en effectuant une activation de la réaction, pour obtenir en particulier des composés de formule générale (Ia), 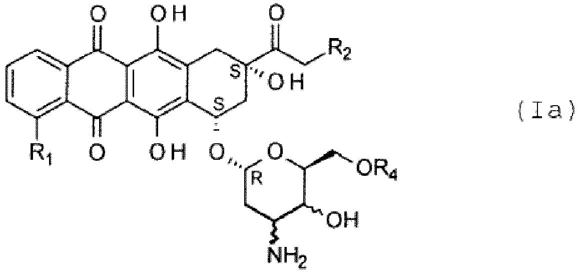 dans laquelle R₁ est un atome d'hydrogène, un groupe hydroxy ou méthoxy, un atome d'halogène ou un groupe NO₂ ; R₂ est un atome d'hydrogène, un groupe hydroxy ou méthoxy, un radical acyle ou aroyle, R₄ représente un groupe (CH₂-CH₂-O)ₙ- où n = 1 à 6, et où un atome d'hydrogène ou un groupe alkyle en C₁-C₄ est attaché à l'atome d'oxygène terminal de la chaîne.

10. Procédé selon la revendication 9, **caractérisé en ce qu'**on prépare des composés de formule (II) en couplant un élément constitutif en C2 de formule générale (V), 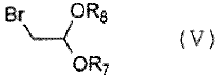 dans laquelle R₇ et R₈ ont la signification donnée dans la formule (II), avec un dérivé protégé du L-thréose (élément constitutif en C4 sous forme d'énantiomère pur) de formule générale (VI), 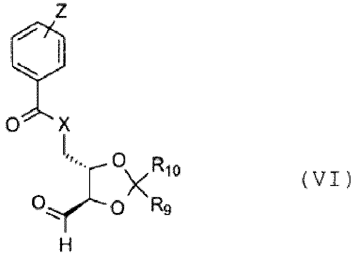 dans laquelle X, R₉, R₁₀ ont la signification donnée dans la formule (II) et Z représente un atome d'hydrogène, ou au moins un ou plusieurs, de préférence un ou deux groupes méthyle, fluoro, chloro, bromo ou nitro, et on oxyde le produit d'addition en résultant, de formule générale (VII) 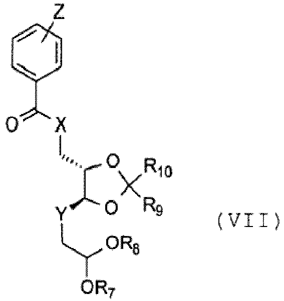 dans laquelle R₇, R₈, R₉, R₁₀ ont la signification donnée dans la formule (II), et Z représente un atome d'hydrogène, ou au moins un ou plusieurs, de préférence un ou deux groupes méthyle, fluoro, chloro, bromo ou nitro et Y représente CH-OH, par oxydation en la cétone [Y = C(=O)] de formule générale (VII) et ensuite on effectue l'introduction de l'azote, de préférence par l'intermédiaire d'une oxime (Y = C(=N)-OH] de formule générale (VII), de laquelle on sépare ensuite le groupe protecteur sur X et on introduit R₄ selon la signification donnée dans la formule (I), en effectuant l'introduction sur X d'une chaîne alkyle ou hétéroalkyle ramifiée ou non ramifiée, par substitution, de préférence à l'aide d'une base non nucléophile, en activant au préalable avec un groupe partant la chaîne alkyle ou hétéroalkyle à introduire et ensuite on réduit à l'aide d'un hydrure métallique l'oxime [Y = C(=N)-OH] de formule générale (II) qui en résulte, pour obtenir des amines [Y = CH-NH₂] de formule générale (II), qui sont ensuite protégées, de sorte qu'il en résulte un composé glucidique de formule générale (II) [Y = CH-NR₅R₆], dans laquelle R₅/R₆ sont différents et ont la signification indiquée dans la formule (I).

11. Composition pharmaceutique comprenant un ou plusieurs composés de formules (I), (Ia) et (IIIc) selon les revendications 2 à 4 et éventuellement une ou plusieurs substances pharmaceutiquement acceptables, choisies parmi des adjuvants, véhicules, diluants et solvants.

12. Nécessaire pharmaceutique comprenant (i) un ou plusieurs composés de formule (I), (Ia) ou (IIIc) selon les revendications 2 à 4, ou une composition pharmaceutique selon la revendication 11 et (ii) au moins une substance active cytotoxique ou antiproliférative.

13. Composés de formule (I), (Ia) ou (IIIc) selon les revendications 2 à 4, composition pharmaceutique selon la revendication 11 ou nécessaire pharmaceutique selon la revendication 12, destinés à l'utilisation en tant que médicament ou en tant qu'outil dans la recherche biomédicale.

14. Composés de formule (I), (Ia) ou (IIIc) selon les revendications 2 à 4, composition pharmaceutique selon la revendication 11 ou nécessaire pharmaceutique selon la revendication 12, destinés à l'utilisation dans le traitement de maladies ou d'états pathologiques, qui peuvent être au moins en partie soulagés par thérapie.

15. Composés de formule (I), (Ia) ou (IIIc) selon les revendications 2 à 4, composition pharmaceutique ou nécessaire pharmaceutique, destinés à l'utilisation selon la revendication 14, dans lesquels les maladies ou états pathologiques sont choisis parmi des maladies prolifératives, de préférence un cancer.
